(19) Europäisches Patentamt / European Patent Office / Office européen des brevets

(11) EP 1 871 735 B1

(12) # EUROPÄISCHE PATENTSCHRIFT

(45) Veröffentlichungstag und Bekanntmachung des Hinweises auf die Patenterteilung:
**23.12.2015 Patentblatt 2015/52**

(21) Anmeldenummer: **06707627.3**

(22) Anmeldetag: **21.03.2006**

(51) Int Cl.:
***C07C 69/734*** (2006.01) ***A61K 8/37*** (2006.01)
***A61K 8/40*** (2006.01) ***A61K 8/41*** (2006.01)
***A61K 8/42*** (2006.01) ***A61Q 17/04*** (2006.01)
***A61Q 19/08*** (2006.01) ***C09K 15/06*** (2006.01)

(86) Internationale Anmeldenummer:
**PCT/EP2006/002592**

(87) Internationale Veröffentlichungsnummer:
**WO 2006/111233 (26.10.2006 Gazette 2006/43)**

(54) **ANTIOXIDANTIEN**
ANTIOXIDANTS
ANTIOXYDANTS

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **19.04.2005 DE 102005018184**
**28.11.2005 EP 05025917**

(43) Veröffentlichungstag der Anmeldung:
**02.01.2008 Patentblatt 2008/01**

(73) Patentinhaber: **Merck Patent GmbH**
**64293 Darmstadt (DE)**

(72) Erfinder:
- **RUDOLPH, Thomas**
**64291 Darmstadt (DE)**
- **BUCHHOLZ, Herwig**
**60599 Frankfurt (DE)**

(56) Entgegenhaltungen:

| | |
|---|---|
| **EP-A- 0 054 174** | **EP-A- 0 100 651** |
| **EP-A- 0 511 666** | **EP-A- 1 266 888** |
| **WO-A-89/09218** | **WO-A-02/094209** |
| **WO-A-03/007906** | **WO-A-03/059864** |
| **DE-A1- 2 035 334** | **US-A- 4 069 340** |
| **US-A- 5 175 340** | **US-A- 5 453 514** |

- **PATENT ABSTRACTS OF JAPAN Bd. 013, Nr. 187 (C-592), 2. Mai 1989 (1989-05-02) & JP 01 013017 A (POLA CHEM IND INC), 17. Januar 1989 (1989-01-17)**
- **IRIE, H. ET AL: "NEW SYNTHESIS OF ISOQUINOLINE ALKALOIDS, THALIFOLINE, CORYPALLINE, AND CHERYLLINE" CHEMISTRY LETTERS, CHEMICAL SOCIETY OF JAPAN. TOKYO, JP, Nr. 7, 1. Juli 1980 (1980-07-01), Seiten 875-878, XP000567042 ISSN: 0366-7022**
- **CHUANG, C-P ET AL: "Manganese(III) Acetate Initiated Oxidative Free Radical Reaction Between 1,4-Naphthoquinones And alpha-Alkylmalonates" TETRAHEDRON, ELSEVIER SCIENCE PUBLISHERS, AMSTERDAM, NL, Bd. 54, Nr. 34, 20. August 1998 (1998-08-20), Seiten 10043-10052, XP004129982 ISSN: 0040-4020**
- **RUSSELL, P.B.; HITCHINGS, G.H.: "Some 2,4,6-Triamino-5-alkyl- and 5-Benzylpyrimidines" J. AM. CHEM. SOC., Bd. 74, Nr. 13, 1952, Seiten 3443-3444, XP002393649**
- **DIANA, G.D. ET AL.: "Antiviral Activity of Some beta-Diketones. 4. Benzyl Diketones. In Vitro Activity against Both RNA and DNA Viruses" J. MED. CHEM., Bd. 21, Nr. 9, 1978, Seiten 889-894, XP002393650**
- **COOK, J.W. ET AL.: "86. Colchicine and Related Compounds. Part III." J. CHEM. SOC., 1944, Seiten 322-325, XP009070577**
- **KUJUNDZIC, N.ET AL.: "Synthesis and Antibacterial Effect of Derivatives of 5-(3,4,5-Trimethoxybenzyl)-pyrimidine, -Tetrahydropyrimidine, -Hexahydropyrimidine and -Hydantoin" CROATICA CHEMICA ACTA, Bd. 61, Nr. 1, 1988, Seiten 121-135, XP009070565**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 871 735 B1

- **JEW, SANG-SUP ET AL: "Enantioselective synthesis of eucomols using Sharpless catalytic asymmetric dihydroxylation" HETEROCYCLES, Bd. 46, 1997, Seiten 65-70, XP009070501**
- **KIRKIACHARIAN, B. SERGE ET AL: "Hydride reduction of coumarin derivatives: new method of synthesis of 2'-hydroxybenzylmalonic esters" COMPTES RENDUS DES SEANCES DE L'ACADEMIE DES SCIENCES, SERIE 2: MECANIQUE-PHYSIQUE, CHIMIE, SCIENCES DE L'UNIVERS, SCIENCES DE LA TERRE, Bd. 294, Nr. 3, 1982, Seiten 181-184, XP009070516**
- **SOHDA, T. ET AL: "Antiulcer activity of 5-benzylthiazolidine-2,4-dione derivatives" CHEMICAL AND PHARMACEUTICAL BULLETIN, PHARMACEUTICAL SOCIETY OF JAPAN, TOKYO, JP, Bd. 31, Nr. 2, Februar 1983 (1983-02), Seiten 560-569, XP002193484 ISSN: 0009-2363**
- **CHRAIBI, A. ET AL.: "Pyrazolidine-diones. I. Synthèse de dihydrazides benzylmaloniques et arylcyclopropaniques" ANNALES PHARMACEUTIQUES FRANÇAISES, Bd. 38, Nr. 4, 1980, Seiten 343-352, XP009070510**
- **HEY, D.H.; NAGDY, K.A.: "Intermolecular acylation. III. The preparation and ring closure of the .alpha.-(methoxyphenyl)glutaric acids" J. CHEM. SOC., 1953, Seiten 1894-1899, XP009070523**
- **BEY, P.; SCHIRLIN, D.: "General approach to the synthesis of alpha-difluoromethyl amines as potential enzyme-activated irreversible inhibitors" TETRAHEDRON LETTERS, Bd. 19, Nr. 52, 1978, Seiten 5225-5228, XP002393651**
- **WESTFAHL, J.C.; GRESHAM, T.L.: "Vinylidene Cyanide. V. The aluminium chloride catalyzed reaction of vinylidene cyanide and aromatic compounds" J. AM. CHEM. SOC., Bd. 76, Nr. 4, 1954, Seiten 1076-1080, XP002393652**
- **DEAN, F.M. ET AL.: "The chemistry of fungi. Part IX. 3,4-Dihydrocoumarins" J. CHEM. SOC., 1950, Seiten 895-902, XP009070552**
- **DATABASE CHEMCATS [Online] Chemical Abstracts Service, Columbus, Ohio, US; XP002393658 gefunden im STN & "Rare Chemicals Catalogue" 27. September 2004 (2004-09-27), RARE CHEMICALS GMBH , 24214 GETTORF, GERMANY**
- **DATABASE CHEMCATS [Online] Chemical Abstracts Service, Columbus, Ohio, US; XP002393659 gefunden im STN & "Rare Chemicals Catalogue" 27. September 2004 (2004-09-27), RARE CHEMICALS GMBH , 24214 GETTORF, GERMANY**


Bemerkungen:

Die Akte enthält technische Angaben, die nach dem Eingang der Anmeldung eingereicht wurden und die nicht in dieser Patentschrift enthalten sind.

## Beschreibung

Die vorliegende Erfindung betrifft die Verwendung von 4-Hydroxy-3,5-dimethoxy-benzyl-malonsäure-di-2-ethylhexylester als Antioxidans bzw. zum Produktschutz, die entsprechende neue Verbindung, sowie das entsprechende Herstellverfahren für diese Verbindung.

Ein Einsatzgebiet der erfindungsgemäßen Verbindung ist beispielsweise die Kosmetik. Aufgabe pflegender Kosmetik ist es, nach Möglichkeit den Eindruck einer jugendlichen Haut zu erhalten. Prinzipiell stehen verschiedene Wege offen, um diesen Weg zu erreichen. So können bereits vorhandene Schädigungen der Haut, wie unregelmäßige Pigmentierung oder Faltenbildung, durch abdeckende Puder oder Cremes ausgeglichen werden. Ein anderer Ansatzpunkt ist, die Haut vor Umwelteinflüssen zu schützen, die zu einer dauerhaften Schädigung und damit Alterung der Haut führen. Die Idee ist also, vorbeugend einzugreifen und dadurch den Alterungsprozess hinauszuzögern. Ein Beispiel sind hierfür UV-Filter, welche durch Absorption bestimmter Wellenlängenbereiche eine Schädigung der Haut vermeiden oder zumindest vermindern. Während bei UV-Filtern das schädigende Ereignis, die UV-Strahlung, von der Haut abgeschirmt wird, versucht man bei einem weiteren Weg, die natürlichen Abwehr- bzw. Reparaturmechanismen der Haut gegen das schädigende Ereignis zu unterstützen. Schließlich verfolgt man als weiteren Ansatzpunkt die mit zunehmendem Alter sich abschwächenden Abwehrfunktionen der Haut gegen schädigende Einflüsse auszugleichen, indem Substanzen von außen zugeführt werden, die diese nachlassende Abwehr- bzw. Reparaturfunktion ersetzen können. Beispielsweise besitzt die Haut die Fähigkeit, Radikale, die durch äußere oder innere Stressfaktoren erzeugt werden, abzufangen. Diese Fähigkeit schwächt sich mit zunehmendem Alter ab, wodurch sich der Alterungsprozess mit zunehmendem Alter beschleunigt.

Eine weitere Schwierigkeit bei der Herstellung von Kosmetika besteht darin, dass Wirkstoffe, die in kosmetische Zubereitungen eingearbeitet werden sollen, oftmals nicht stabil sind und in der Zubereitung geschädigt werden können. Die Schädigungen können beispielsweise durch eine Reaktion mit Luftsauerstoff oder durch die Absorption von UV-Strahlen verursacht werden. Die so geschädigten Moleküle können durch ihre Strukturänderung z.B. ihre Farbe ändern und/oder ihre Wirksamkeit verlieren. Entsprechende Schwierigkeiten treten allgemein bei der Herstellung, Lagerung oder Anwendung von Zubereitungen enthaltend oxidationsempfindliche Inhaltsstoffe auf.

Ein bekannter Weg, die beschriebenen Probleme zu behandeln besteht im Zusatz von Antioxidantien zu den Zubereitungen.

Laut CD Römpp Chemie Lexikon - Version 1.0, Stuttgart/New York: Georg Thieme Verlag 1995 handelt es sich bei Antioxidantien um Verbindungen, die unerwünschte, durch Sauerstoff-Einwirkungen u.a. oxidative Prozesse bedingte Veränderungen in den zu schützenden Stoffen hemmen oder verhindern. Einsatzgebiete sind z.B. in Kunststoffen und Kautschuk zum Schutz gegen Alterung; in Fetten zum Schutz vor Ranzigkeit, in Ölen, Viehfutter, Autobenzin und Düsentreibstoffen zum Schutz gegen Verharzung, in Transformatoren- und Turbinenöl gegen Schlammbildung, in Aromastoffen gegen Geruchsverschlechterung. Als Antioxidantien wirksam sind u.a. durch sterisch hindernde Gruppen substituierte Phenole, Hydrochinone, Brenzcatechine, Aromaten, Amine sowie deren MetallKomplexe. Die Wirkung der Antioxidantien besteht laut Römpp meist darin, daß sie als Radikalfänger für die bei der Autoxidation auftretenden freien Radikale wirken.

In WO 03/007906 A sind strukturell ähnliche Verbindungen offenbart, die als wesentliches Merkmal jedoch eine Doppelbindung enthalten.

Die Zusammenfassung der japanischen Offenlegungsschrift JP 01013017 beschreibt 3,5-Dimethoxy-4-hydroxyzimtsäurederivate, deren wesentliches Merkmal eine Doppelbindung ist.

EP 0 100 651 A beschreibt strukturell ähnliche Dialkylmalonate als organische Sonnenschutzmittelzusatz, deren wesentliches Merkmal jedoch eine Doppelbindung ist.

US 5,175,340 beschreibt ungesättigte Benzalmalonate, deren wesentliches Merkmal eine Doppelbindung ist.

Es besteht jedoch weiterhin Bedarf nach hautverträglichen Antioxidantien, die sich auch zum Einsatz in hautpflegenden Zubereitungen eignen.

Aufgabe der Erfindung ist es daher, eine Verbindung zur Verfügung zu stellen, welche eine schützende Wirkung gegen oxidativen Stress auf Körperzellen ausübt und/oder einer Alterung der Haut entgegenwirkt.

Ein erster Gegenstand der vorliegenden Erfindung ist daher die Verwendung der Verbindung 4-Hydroxy-3,5-dimethoxy-benzyl-malonsäure-di-2-ethylhexylester als Antioxidans.

Die beschriebene Verbindung kann erfindungsgemäß als Antioxidans zur topischen Anwendung bzw. zur Herstellung kosmetischer oder dermatologischer Zubereitungen oder zur Herstellung von Haushaltsprodukten verwendet werden. Die beschriebene Verbindung kann zum Produktschutz eingesetzt werden. Produktschutz bedeutet dabei im Sinne dieser Anmeldung insbesondere den Schutz oxidationsempfindlicher Formulierungsbestandteile wie organische oder anorganische Farbstoffe, Antioxidantien, Vitamine, Parfumkomponenten, Ölkomponenten oder Matrixbestandteile, wie Emulgatoren, Verdicker, Filmbildner und Tenside. Die entsprechende Verwendung ist Gegenstand dieser Anmeldung.

Erfindungsgegenstand ist auch die Verwendung der Verbindung 4-Hydroxy-3,5-dimethoxy-benzyl-malonsäure-di-2-ethylhexylester als Antioxidans zur Herstellung kosmetischer oder dermatologischer Zubereitungen oder zur Herstellung von Haushaltsprodukten.

Ein weiterer Gegenstand der vorliegenden Erfindung ist die Verbindung 4-Hydroxy-3,5-dimethoxy-benzyl-malonsäure-di-2-ethylhexyl-ester.

Die Verbindung zeigt eine besonders ausgeprägte antioxidative Leistung.

Insbesondere bevorzugt ist dabei die Verwendung von 4-Hydroxy-3,5-dimethoxy-benzyl-malonsäure-di-2-ethyl-hexylester.

Für die erfindungsgemäße Verwendung sind Zubereitungen geeignet. Bei den Zubereitungen handelt es sich dabei üblicherweise entweder um topisch anwendbare Zubereitungen, beispielsweise kosmetische oder dermatologische Formulierungen, oder um Haushaltsprodukte. Die Zubereitungen enthalten in diesem Fall einen kosmetisch oder dermatologisch oder Haushaltsprodukte-geeigneten Träger und je nach gewünschtem Eigenschaftsprofil optional weitere geeignete Inhaltsstoffe. Vorteile der erfindungsgemäßen Verbindung bzw. Verwendung der erfindungsgemäßen Verbindung können dabei insbesondere sein:

- eine antioxidante Wirkung gegen Radikale, die z.B. durch UV-Licht oder durch thermolytische Prozesse, wie dem Rauchen, induziert werden, wie z.B. gegen das Superoxidradikalanion oder das NO-Radikal, bzw. gegen reaktive Sauerstoffspezies, wie z.B. gegen Singuletsauerstoff und Peroxide,
- bevorzugte Verbindung vereinigt in sich eine starke antioxidante Aktivität in Kombination mit einer hohen molekularen Stabilität,
- eine produktstabilisierende Wirkung auf kosmetische, pharmazeutische, insbesondere dermatologische Produkte oder Haushaltsprodukte bzw. Nahrungsmittel und Nahrungsergänzungsmittel, insbesondere solche, die Farb-, Konsistenz- oder Geruchsstoffe enthalten,
- bevorzugte Verbindung eignet sich als Ölkomponente in Zubereitungen,
- bevorzugte Verbindung eignet sich zur Verbesserung von galenischen Eigenschaften, wie beispielsweise des Hautgefühls, von Zubereitungen,
- bevorzugte Verbindung zeigt gute Löslichkeits- und Lösungsmitteleigenschaften, vorzugsweise z.B. als Lösungsmittel für kristalline Komponenten,
- die gute Hautverträglichkeit,
- eine produktstabilisierende Wirkung auf Pigmente und Lacke,
- bevorzugte Verbindung eignet sich zur Erzeugung bzw. Erhöhung ("boost"-Effekt) von Lichtschutzfaktoren, wie LSF, SPF, PPD oder IPD, oder Radikalschutzfaktoren,
- eine stabilisierende Wirkung auf autooxidierbare Polyethylenglycol (PEG)- oder Polyglycerin (PG)-Derivate, wie insbesondere PEG- oder PG-haltige Emulgatoren, wie sie weiter unten in dieser Anmeldung genannt werden, bzw. eine Reduktion der schädigenden Wirkung der Abbauprodukte autooxidierbarer Polyethylenglycol (PEG)- oder Polyglycerin (PG)-Derivate,
- eine stabilisierende Wirkung auf Farb-, Konsistenz- oder Geruchsstoffe, oder auf Antioxidantien oder Vitamine, und UV Filter sowie Titandioxidhaltige Pigmente, insbesondere in kosmetische, pharmazeutische, insbesondere dermatologische Produkten oder Haushaltsprodukten bzw. Nahrungsmitteln und Nahrungsergänzungsmitteln,
- während die meisten Antioxidantien nach Reaktion mit Radikalen wirkungslos werden, zeigt bevorzugte Verbindung nach dieser Reaktion UV-filternde Wirkung und setzt so ihre Schutzfunktion fort,
- bevorzugte erfindungsgemäße Verbindung mit antioxidanten Eigenschaften kann auch zur Pigmentierungskontrolle eingesetzt werden, da sie eine aufhellende Wirkung auf Hautpartien haben kann.

Zusätzlich ist die hier beschriebene Verbindung farblos oder nur schwach gefärbt und führt so nicht oder nur in geringer Weise zu Verfärbungen der Zubereitungen.

Bevorzugt kann es dabei sein, wenn die Zubereitung mindestens die Verbindung 4-Hydroxy-3,5-dimethoxy-benzyliden-malonsäure-di-2-ethyl-hexylester, enthält.

Die Verbindung wird erfindungsgemäß typisch in Mengen von 0,01 bis 20 Gew.-%, vorzugsweise in Mengen von 0,1 Gew.-% bis 10 Gew.-% und insbesondere bevorzugt in Mengen von 1 bis 8 Gew.-% eingesetzt. Dabei bereitet es dem Fachmann keinerlei Schwierigkeiten die Mengen abhängig von der beabsichtigten Wirkung der Zubereitung entsprechend auszuwählen.

Damit die erfindungsgemäße Verbindung ihre positive Wirkung als Radikalfänger auf die Haut besonders gut entwickeln kann, kann es bevorzugt sein die erfindungsgemäße Verbindung in tiefere Hautschichten eindringen zu lassen. Dazu stehen mehrere Möglichkeiten zur Verfügung. Zum einen kann die erfindungsgemäße Verbindung eine ausreichende Lipophilie aufweisen, um durch die äußere Hautschicht in epidermale Schichten vordringen zu können. Als weitere Möglichkeit können in der Zubereitung auch entsprechende Transportmittel, beispielsweise Liposomen, vorgesehen sein, die einen Transport der erfindungsgemäßen Verbindungen durch die äußeren Hautschichten ermöglichen. Schließlich ist auch ein systemischer Transport der erfindungsgemäßen Verbindung denkbar. Die Zubereitung

wird dann beispielsweise so gestaltet, dass sie für eine orale Gabe geeignet ist.

Allgemein wirkt die Substanz als Radikalfänger. Solche Radikale werden exogen nicht nur durch Sonnenlicht erzeugt, sondern auch durch die Einwirkung reaktiver Substanzen, wie Ozon, Stickoxiden (z. B. Zigarettenrauch) oder Schwermetallbelastung (z. B. in der Nahrung). Weitere Beispiele sind Anoxie, die den Elektronenfluß stromauf der Cytochromoxidasen blockiert und die Bildung von Superoxidradikalarionen bedingt; Entzündungen, die unter anderem mit der Bildung von Superoxidanionen durch die Membran-NADPH-Oxidase der Leukozyten einhergehen, die jedoch auch mit der Bildung (durch Disproportionierung in Gegenwart von Eisen (II)-ionen) der Hydroxyradikale und anderer reaktiver Spezies, die normalerweise beim Phänomen einer Phagocytose beteiligt sind, einhergehen; sowie Lipidautooxidation die im Allgemeinen durch ein Hydroxylradikal initiiert wird und lipidische Alkoxyradikale und Hydroperoxide liefert.

Aufgrund dieser Eigenschaften eignet sich die erfindungsgemäße Verbindung allgemein zur Immunprotektion und zum Schutz der DNA und RNA. Insbesondere eignet sich die Verbindung dabei zum Schutz von DNA und RNA vor oxidativen Angriffen, vor Radikalen und vor Schädigung durch Strahlung, insbesondere UV-Strahlung. Ein weiterer Vorteil der erfindungsgemäßen Verbindung ist der Zellschutz, insbesondere der Schutz von Langerhans-Zellen vor Schäden durch die oben genannten Einflüsse.

Insbesondere eignet sich die erfindungsgemäße Verbindung auch zur Behandlung von Hautkrankheiten, die mit einer Störung der Keratinisierung verbunden sind, die die Differenzierung und Zellprolif-eration betrifft, insbesondere zur Behandlung der Akne vulgaris, Akne comedonicä, der polymorphen Akne, der Akne rosaceae, der nodulären Akne, der Akne conglobata, der alters-bedingten Aknen, der als Neben-wirkung auftretenden Aknen, wie der Akne solaris, der medikamenten-bedingten Akne oder der Akne professionalis, zur Behandlung anderer Störungen der Keratinisierung, insbesondere der Ichtyosen, der ichtyosi-formen Zustände, der Darrier-Krankheit, der Keratosis palmoplantaris, der Leukoplasien, der leukoplasiformen Zustände, der Haut- und Schleimhaut-flechten (Buccal) (Lichen), zur Behandlung anderer Hauterkrankungen, die mit einer Störung der Keratinisierung zusammenhängen und eine entzünd-liche und/oder immunoallergische Komponente haben und insbesondere aller Formen der Psoriasis, die die Haut, die Schleimhäute und die Finger und Zehennägel betreffen, und des psoriatischen Rheumas und der Haut-atopien, wie Ekzemen oder der respiratorischen Atopie oder auch der Hypertrophie des Zahnfleisches, wobei die Verbindungen ferner bei einigen Entzündungen verwendet werden können, die nicht mit einer Störung der Keratinisierung zusammenhängen, zur Behandlung aller gutartigen oder bösartigen Wucherungen der Dermis oder Epidermis, die gegebenenfalls viralen Ursprungs sind, wie Verruca vulgaris. Veruca plana, Epidermodysplasia verruciformis, orale Papillomatose, Papillo-matosis florida, und der Wucherungen, die durch UV-Strahlung hervor-gerufen werden können, insbesondere des Epithelioma baso-cellulare und Epithelioma spinocellulare, zur Behandlung anderer Hautkrankheiten, wie der Dermatitis bullosa und der das Kollagen betreffenden Krankheiten, zur Behandlung bestimmter Augenkrankheiten, insbesondere der Hornhauterkran-kungen, zur Behebung oder Bekämpfung der lichtbedingten und der mit dem Älterwerden zusammenhängenden Hautalterung, zur Verminderung der Pigmentierungen und der Keratosis actinica und zur Behandlung aller Krankheiten, die mit der normalen Alterung oder der lichtbedingten Alterung zusammenhängen, zur Vorbeugung vor oder der Heilung von Wunden/Narben der Atrophien der Epidermis und/oder Dermis, die durch lokal oder systemisch angewendete Corticosteroide hervorgerufen werden und aller sonstigen Arten der Hautatrophie, zur Vorbeugung vor oder Behandlung von Störungen der Wundheilung, zur Vermeidung oder Behebung von Schwanger-schaftsstreifen oder auch zur Förderung der Wundheilung, zur Bekämpfung von Störungen der Talgproduktion, wie Hypersebhorrhö bei Akne oder der einfachen Seborrhö, zur Bekämpfung von oder Vorbeugung von krebsartigen Zuständen oder vor präkanzerogenen Zuständen, insbesondere der promyelozytären Leukämien, zur Behandlung von Entzündungserkrankungen, wie Arthritis, zur Behandlung aller virusbedingten Erkrankungen der Haut oder anderer Bereiche des Körpers, zur Vorbeugung vor oder Behandlung der Alopecie, zur Behandlung von Hautkrankheiten oder Krankheiten anderer Körperbereiche mit einer immuno-logischen Komponente, zur Behandlung von Herz-/Kreislauf-Erkran-kungen, wie Arteriosklerose oder Bluthochdruck, sowie des Insulin-unabhängigen Diabetes, zur Behandlung von Hautproblemen, die durch UV-Strahlung hervorgerufen werden.

Die Antioxidante Wirkung der erfindungsgemäßen Verbindung kann beispielsweise mit dem 2,2-Diphenyl-1-picrylhydrazyl(DPPH)-Assay gezeigt werden. 2,2-Diphenyl-1-picrylhydrazyl ist ein in Lösung stabiles freies Radikal. Das ungepaarte Elektron führt zu einer starken Absorptionsbande bei 515 nm, die Lösung ist dunkel-violett gefärbt. In Gegenwart eines Radikalfängers wird das Elektron gepaart, die Absorption verschwindet und die Entfärbung verläuft stöchiometrisch unter Berücksichtigung der aufgenommenen Elektronen. Gemessen wird die Extinktion im Photometer. Die antiradikalische Eigenschaft der zu testenden Substanz wird bestimmt, indem man die Konzentration ermittelt, bei der 50 % des eingesetzten 2,2-Diphenyl-1-picrylhydrazyls mit dem Radikalfänger reagiert haben. Ausgedrückt wird diese Konzentration als $EC_{50}$, ein Wert, der unter den gegebenen Messbedingungen als Substanzeigenschaft zu betrachten ist. Verglichen wird die untersuchte Substanz mit einem Standard (z.B. Tocopherol). Der $EC_{50}$-Wert ist dabei ein Maß für die Kapazität der jeweiligen Verbindung Radikale zu fangen. Je niedriger der $EC_{50}$-Wert ist, desto höher ist die Kapazität Radikale zu fangen. Im Sinne dieser Erfindung wird von einer großen oder hohen Kapazität Radikale zu fangen gesprochen, wenn der $EC_{50}$-Wert niedriger als der von Tocopherol.

Ein weiterer wichtiger Aspekt für die Wirkung der Antioxidantien ist die Zeit in der dieser $EC_{50}$-Wert erreicht wird. Diese Zeit gemessen in Minuten ergibt den $T_{EC50}$-Wert, der eine Aussage über die Geschwindigkeit zulässt, mit der diese Antioxidantien Radikale fangen. Im Sinne dieser Erfindungen gelten Antioxidantien, die diesen Wert innerhalb von weniger als 60 Minuten erreichen als schnell, solche die den $EC_{50}$-Wert erst nach mehr als 120 Minuten erreichen als zeitverzögert wirkend.

Die antiradikalische Effizienz (AE) (beschrieben bei C. Sanchez-Moreno, J.A. Larrauri und F. Saura-Calixto in J. Sci. Food Agric. 1998, 76(2), 270-276.) ergibt sich aus den oben genannten Größen nach folgender Beziehung:

$$AE = \frac{1}{EC_{50} T_{EC50}}$$

Eine niedrige AE (x$10^3$) liegt im Bereich bis etwa 10, von einer mittleren AE wird im Bereich von 10 bis 20 gesprochen und eine hohe AE liegt erfindungsgemäß bei Werten oberhalb 20 vor.

Dabei kann es insbesondere bevorzugt sein, schnell wirkende Antioxidantien mit solchen mit langsamer oder zeitverzögerter Wirkung zu kombinieren. Dabei sind typische Gewichtsverhältnisse der schnell wirkenden Antioxidantien zu zeitverzögert wirkenden Antioxidantien im Bereich 10:1 bis 1:10, vorzugsweise im Bereich 10:1 bis 1:1 und für hautschützende Zubereitungen insbesondere bevorzugt im Bereich 5:1 bis 2:1. In anderen bevorzugten Zubereitungen kann es im Sinne einer Wirkungsoptimierung allerdings von Vorteil sein, mehr zeitverzögert wirkende Antioxidantien als schnell wirkende Antioxidanten vorliegen. Typische Zusammensetzungen zeigen dann Gewichtsverhältnisse der schnell wirkenden Antioxidantien zu zeitverzögert wirkenden Antioxidantien im Bereich 1:1 bis 1:10, vorzugsweise im Bereich 1:2 bis 1:8.

Die schützende Wirkung gegen oxidativen Stress bzw. gegen die Einwirkung von Radikalen kann also weiter verbessert werden, wenn die Zubereitungen ein oder mehrere weitere Antioxidantien enthalten, wobei es dem Fachmann keinerlei Schwierigkeiten bereitet geeignet schnell oder zeitverzögert wirkende Antioxidantien auszuwählen.

In einer bevorzugten Ausführungsform handelt es sich bei der Zubereitung daher um eine Zubereitung zum Schutz von Körperzellen gegen oxidativen Stress, insbesondere zur Verringerung der Hautalterung, die neben 4-Hydroxy-3,5-dimethoxy-benzyl-malonsäure-di-2-ethylhexylester vorzugsweise ein oder mehrere weitere Antioxidantien enthält.

Es gibt viele aus der Fachliteratur bekannte und bewährte Substanzen, die als Antioxidantien verwendet werden können, z.B. Aminosäuren (z.B. Glycin, Histidin, Tyrosin, Tryptophan) und deren Derivate, Imidazole, (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis μmol/kg), ferner (Metall-) Chelatoren, (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate, Vitamin C und Derivate (z.B. Ascorbylpalmitat, Magnesium-Ascorbylphosphat, Ascorbylacetat), Tocopherole und Derivate (z.B. Vitamin-E-acetat), Vitamin A und Derivate (z.B. Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordohydroguajaretsäure, Trihydroxybutyrophenon, Quercitin, Harnsäure und deren Derivate, Mannose und deren Derivate, Zink und dessen Derivate (z.B. ZnO, $ZnSO_4$), Selen und dessen Derivate (z.B. Selenmethionin), Stilbene und deren Derivate (z.B. Stilbenoxid, trans-Stilbenoxid).

Mischungen von Antioxidantien sind ebenfalls zur Verwendung in den kosmetischen Zubereitungen geeignet. Bekannte und käufliche Mischungen sind beispielsweise Mischungen enthaltend als aktive Inhaltsstoffe Lecithin, L-(+)-Ascorbylpalmitat und Zitronensäure (z.B. (z.B. Oxynex® AP), natürliche Tocopherole, L-(+)-Ascorbylpalmitat, L-(+)-Ascorbinsäure und Zitronensäure (z.B. Oxynex® K LIQUID), Tocopherolextrakte aus natürlichen Quellen, L-(+)-Ascorbylpalmitat, L-(+)-Ascorbinsäure und Zitronensäure (z.B. Oxynex® L LIQUID), DL-α-Tocopherol, L-(+)-Ascorbylpalmitat, Zitronensäure und Lecithin (z.B. Oxynex® LM) oder Butylhydroxytoluol (BHT), L-(+)-Ascorbylpalmitat und Zitronensäure (z.B. Oxynex® 2004). Derartige Antioxidantien werden mit der erfindungsgemäßen Verbindung in solchen Zusammensetzungen überlicherweise in Verhältnissen im Bereich von 1000:1 bis 1:1000, bevorzugt in Mengen von 100:1 bis 1:100 eingesetzt.

Die Zubereitungen können als weitere Inhaltsstoffe Vitamine enthalten. Bevorzugt sind Vitamine und Vitamin-Derivate ausgewählt aus Vitamin A, Vitamin-A-Propionat, Vitamin-A-Palmitat, Vitamin-A-Acetat, Retinol, Vitamin B, Thiaminchloridhydrochlorid (Vitamin $B_1$), Riboflavin (Vitamin $B_2$), Nicotinsäureamid, Vitamin C (Ascorbinsäure), Vitamin D, Ergocalciferol (Vitamin $D_2$), Vitamin E, DL-$\alpha$-Tocopherol, Tocopherol-E-Acetat, Tocopherolhydrogensuccinat, Vitamin $K_1$, Esculin (Vitamin P-Wirkstoff), Thiamin (Vitamin $B_1$), Nicotinsäure (Niacin), Pyridoxin, Pyridoxal, Pyridoxamin, (Vitamin $B_6$), Panthothensäure, Biotin, Folsäure und Cobalamin (Vitamin $B_{12}$) in den erfindungsgemäßen kosmetischen Zubereitungen enthalten, insbesondere bevorzugt Vitamin-A-Palmitat, Retinol, Vitamin C und dessen Derivaten, DL-$\alpha$-Tocopherol, Tocopherol-E-Acetat, Nicotinsäure, Pantothensäure und Biotin. Vitamine werden dabei mit der erfindungsgemäßen Verbindung üblicherweise in Verhältnissen im Bereich von 1000:1 bis 1:1000, bevorzugt in Mengen von 100:1 bis 1:100 eingesetzt.

Dabei hat sich gezeigt, dass Antioxidantien, wie beispielsweise beta-Carotin und Tocopherol die Konversion der erfindungsgemäßen Verbindung zur UV-filternden Verbindung beschleunigen können.

Die erfindungsgemäß einzusetzende Verbindung weist - nach Bestrahlung - eine UV-Absorption im UV-A- und oder UV-B-Bereich auf. Bevorzugte Zubereitungen können auch als Sonnenschutzmittel dienen und enthalten dann neben den erfindungsgemäßen Verbindungen auch UV-Filter.

Bei Einsatz der insbesondere als UV-A-Filter bevorzugten, aber auch als UVB-Filter verwendeten Dibenzoylmethanderivate, oder der insbesondere als UVB-Filter eingesetzten Zimtsäurederivate in Kombination mit der erfindungsgemäßen Verbindung ergibt sich ein zusätzlicher Vorteil: Die UV-empfindlichen Dibenzoylmethanderivate und Zimtsäurederivate werden durch die Anwesenheit der erfindungsgemäßen Verbindungen zusätzlich stabilisiert.

Prinzipiell kommen alle UV-Filter für eine Kombination mit der erfindungsgemäßen Verbindung in Frage. Besonders bevorzugt sind solche UV-Filter, deren physiologische Unbedenklichkeit bereits nachgewiesen ist. Sowohl für UVA wie auch UVB-Filter gibt es viele aus der Fachliteratur bekannte und bewährte Substanzen, z.B.

Benzylidenkampferderivate wie 3-(4'-Methylbenzyliden)-dl-kampfer (z.B. Eusolex® 6300), 3-Benzylidenkampfer (z.B. Mexoryl® SD), Polymere von N-{(2 und 4)-[(2-oxoborn-3-yliden)methyl]benzyl}-acrylamid (z.B. Mexoryl® SW), N,N,N-Trimethyl-4-(2-oxoborn-3-ylidenmethyl)anilinium methylsulfat (z.B. Mexoryl® SK) oder (2-Oxoborn-3-yliden)toluol-4-sulfonsäure (z.B. Mexoryl® SL),

Benzoyl- oder Dibenzoylmethane wie 1-(4-tert-Butylphenyl)-3-(4-methoxyphenyl)propan-1,3-dion (z.B. Eusolex® 9020) oder 4-Isopropyldibenzoylmethan (z.B. Eusolex® 8020),

Benzophenone wie 2-Hydroxy-4-methoxybenzophenon (z.B. Eusolex® 4360) oder 2-Hydroxy-4-methoxybenzophenon-5-sulfonsäure und ihr Natriumsalz (z.B. Uvinul® MS-40),

Methoxyzimtsäureester wie Methoxyzimtsäureoctylester (z.B. Eusolex® 2292), 4-Methoxyzimtsäureisopentylester, z.B. als Gemisch der Isomere (z.B. Neo Heliopan® E 1000),

Salicylatderivate wie 2-Ethylhexylsalicylat (z.B. Eusolex® OS), 4-Isopropylbenzylsalicylat (z.B. Megasol®) oder 3,3,5-Trimethylcyclohexylsalicylat (z.B. Eusolex® HMS), 4-Aminobenzoesäure und Derivate wie 4-Aminobenzoesäure, 4-(Dimethylamino)benzoesäure-2-ethylhexylester (z.B. Eusolex® 6007), ethoxylierter 4-Aminobenzoesäureethylester (z.B. Uvinul® P25),

Phenylbenzimidazolsulfonsäuren, wie 2-Phenylbenzimidazol-5-sulfonsäure sowie ihre Kalium-, Natrium- und Triethanolaminsalze (z.B. Eusolex® 232), 2,2-(1,4-Phenylen)-bisbenzimidazol-4,6-disulfonsäure bzw. deren Salze (z.B. Neoheliopan® AP) oder 2,2-(1,4-Phenylen)-bisbenzimidazol-6-sulfonsäure;
und weitere Substanzen wie

- 2-Cyano-3,3-diphenylacrylsäure-2-ethylhexylester (z.B. Eusolex® OCR),
- 3,3'-(1,4-Phenylendimethylen)-bis-(7,7-dimethyl-2-oxobicyclo-[2.2.1]hept-1-ylmethansulfonsäure sowie ihre Salze (z.B. Mexoryl® SX) und
- 2,4,6-Trianilino-(p-carbo-2'-ethylhexyl-1'-oxi)-1,3,5-triazin (z.B. Uvinul® T 150)
- 2-(4-Diethylamino-2-hydroxy-benzoyl)-benzoesäure hexylester (z.B. Uvinul®UVA Plus, Fa. BASF).

Die in der Liste aufgeführten Verbindungen sind nur als Beispiele aufzufassen. Selbstverständlich können auch andere UV-Filter verwendet werden.

Diese organischen UV-Filter werden in der Regel in einer Menge von 0,5 bis 10 Gewichtsprozent, vorzugsweise 1 - 8 %, in kosmetische Formulierungen eingearbeitet.

Weitere geeignete organische UV-Filter sind z.B.

- 2-(2H-Benzotriazol-2-yl)-4-methyl-6-(2-methyl-3-(1,3,3,3-tetramethyl-1-(trimethylsilyloxy)disiloxanyl)propyl)phenol (z.B. Silatrizole®),
- 4,4'-[(6-[4-((1,1-Dimethylethyl)aminocarbonyl)phenylamino]-1,3,5-triazin-2,4-diyl)diimino]bis(benzoesäure-2-ethylhexylester) (z.B. Uvasorb® HEB),
- $\alpha$-(Trimethylsilyl)-$\omega$-[trimethylsilyl)oxy]poly[oxy(dimethyl [und ca. 6% methyl[2-[p-[2,2-bis(ethoxycarbonyl]vinyl]phenoxy]-1-methylenethyl] und ca. 1,5 % methyl[3-[p-[2,2-bis(ethoxycarbonyl)vinyl)phenoxy)-propenyl) und 0,1

bis 0,4% (methylhydrogen]silylen]] (n ≈ 60) (CAS-Nr. 207 574-74-1)

- 2,2'-Methylen-bis-(6-(2H-benzotriazol-2-yl)-4-(1,1,3,3-tetramethylbutyl)phenol) (CAS-Nr. 103 597-45-1)
- 2,2'-(1,4-Phenylen)bis-(1 H-benzimidazol-4,6-disulfonsäure, Mononatriumsalz) (CAS-Nr. 180 898-37-7) und
- 2,4-bis-{[4-(2-Ethyl-hexyloxy)-2-hydroxyl]-phenyl}-6-(4-methoxyphenyl)-1,3,5-triazin (CAS-Nr. 103 597-45-, 187 393-00-6).
- 4,4'-[(6-[4-((1,1-Dimethylethyl)aminocarbonyl)phenylamino]-1,3,5-triazin-2,4-diyl)diimino]bis(benzoesäure-2-ethylhexylester) (z.B. Uvasorb® HEB),

Weitere geeignete UV-Filter sind auch Methoxyflavone ensprechend der älteren Deutschen Patentanmeldung DE-A-10232595.

Organische UV-Filter werden in der Regel in einer Menge von 0,5 bis 20 Gewichtsprozent, vorzugsweise 1 - 15 %, in kosmetische Formulierungen eingearbeitet.

Als anorganische UV-Filter sind solche aus der Gruppe der Titandioxide wie z.B. gecoatetes Titandioxid (z.B. Eusolex® T-2000, Eusolex®T-AQUA; Eusolex® T-AVO), Zinkoxide (z.B. Sachtotec®), Eisenoxide oder auch Ceroxide denkbar. Diese anorganischen UV-Filter werden in der Regel in einer Menge von 0,5 bis 20 Gewichtsprozent, vorzugsweise 2 - 10 %, in kosmetische Zubereitungen eingearbeitet.

Bevorzugte Verbindungen mit UV-filternden Eigenschaften sind 3-(4'-Methylbenzyliden)-dl-kampfer, 1-(4-tert-Butylphenyl)-3-(4-methoxy-phenyl)-pro-pan-1,3-dion, 4-Isopropyldibenzoylmethan, 2-Hydroxy-4-meth-oxy-ben-zo--phenon, Methoxyzimtsäureoctylester, 3,3,5-Trimethyl-cyclo-hexyl-sali-cylat, 4-(Dimethylamino)benzoesäure-2-ethylhexylester, 2-Cyano-3,3-di-phenyl-acrylsäure-2-ethylhexylester, 2-Phenyl-benzimidazol-5-sulfonsäure sowie ihre Kalium-, Natrium- und Triethanol-aminsalze.

Durch Kombination der erfindungsgemäßen Verbindung mit weiteren UV-Filtern kann die Schutzwirkung gegen schädliche Einwir-kungen der UV-Strahlung optimiert werden.

Optimierte Zusammensetzungen können beispielsweise die Kombination der organischen UV-Filter 4'-Methoxy-6-hydroxyflavon mit 1-(4-tert-Butylphenyl)-3-(4-methoxyphenyl)propan-1,3-dion und 3-(4'-Methylbenzyliden)-dl-kampfer enthalten. Mit dieser Kombination ergibt sich ein Breitbandschutz, der durch Zusatz von anorganischen UV-Filtern, wie Titandioxid-Mikropartikeln noch ergänzt werden kann.

Alle genannten UV-Filter und die erfindungsgemäßen Verbindungen können auch in verkapselter Form eingesetzt werden. Insbesondere ist es von Vorteil organische UV-Filter in verkapselter Form einzusetzen. Im Einzelnen ergeben sich die folgende Vorteile:

- Die Hydrophilie der Kapselwand kann unabhängig von der Löslichkeit des UV-Filters bzw. der Verbindung der Formel I eingestellt werden. So können beispielsweise auch hydrophobe UV-Filter bzw. erfindungsgemäßen Verbindungen in rein wässrige Zubereitungen eingearbeitet werden. Zudem wird der häufig als unangenehm empfundene ölige Eindruck beim Auftragen der hydrophobe UV-Filter enthaltenden Zubereitung unterbunden.
- Bestimmte UV-Filter, insbesondere Dibenzoylmethanderivate, zeigen in kosmetischen Zubereitungen nur eine verminderte Photostabilität. Durch Verkapselung dieser Filter oder von Verbindungen, welche die Photostabilität dieser Filter beeinträchtigen, wie beispielsweise Zimtsäurederivate, kann die Photostabilität der gesamten Zubereitung erhöht werden.
- In der Literatur wird immer wieder die Hautpenetration durch organische UV-Filter und das damit verbundene Reizpotential beim direkten Auftragen auf die menschliche Haut diskutiert. Durch die hier vorgeschlagene Verkapselung der entsprechenden Substanzen wird dieser Effekt unterbunden.
- Allgemein können durch Verkapselung einzelner UV-Filter bzw. erfindungsgemäßen Verbindungen oder anderer Inhaltstoffe Zubereitungsprobleme, die durch Wechselwirkung einzelner Zubereitungsbestandteile untereinander entstehen, wie Kristallisationsvorgänge, Ausfällungen und Agglomeratbildung vermieden werden, da die Wechselwirkung unterbunden wird.

Daher ist es bevorzugt, wenn ein oder mehrere der oben genannten UV-Filter bzw. die erfindungsgemäße Verbindung in verkapselter Form vorliegen. Vorteilhaft ist es dabei, wenn die Kapseln so klein sind, dass sie mit dem bloßen Auge nicht beobachtet werden können. Zur Erzielung der o.g. Effekte ist es weiterhin erforderlich, dass die Kapseln hinreichend stabil sind und den verkapselten Wirkstoff (UV-Filter) nicht oder nur in geringem Umfang an die Umgebung abgeben.

Geeignete Kapseln können Wände aus anorganischen oder organischen Polymeren aufweisen. Beispielsweise wird in US 6,242,099 B1 die Herstellung geeigneter Kapseln mit Wänden aus Chitin, Chitin-Derivaten oder polyhydroxylierten Polyaminen beschrieben. Erfindungsgemäß besonders bevorzugt einzusetzende Kapseln weisen Wände auf, die durch einen SolGel-Prozeß, wie er in den Anmeldungen WO 00/09652, WO 00/72806 und WO 00/71084 beschrieben ist, erhalten werden können. Bevorzugt sind hier wiederum Kapseln, deren Wände aus Kieselgel (Silica; undefiniertes Silicium-oxid-hydroxid) aufgebaut sind. Die Herstellung entsprechender Kapseln ist dem Fachmann beispielsweise aus

den zitierten Patentanmeldungen bekannt, deren Inhalt ausdrücklich auch zum Gegenstand der vorliegenden Anmeldung gehört.

Dabei sind die Kapseln in den Zubereitungen vorzugsweise in solchen Mengen enthalten, die gewährleisten, dass die verkapselten UV-Filter in den oben angegebenen Mengen in der Zubereitung vorliegen.

Die Zubereitungen können darüber hinaus weitere übliche hautschonende oder hautpflegende Wirkstoffe enthalten. Dies können prinzipiell alle den Fachmann bekannten Wirkstoffe sein.

Besonders bevorzugte Wirkstoffe sind Pyrimidincarbonsäuren und/oder Aryloxime.

Pyrimidincarbonsäuren kommen in halophilen Mikroorganismen vor und spielen bei der Osmoregulation dieser Organismen eine Rolle (E. A. Galinski et al., Eur. J. Biochem., 149 (1985) Seite 135-139). Dabei sind unter den Pyrimidincarbonsäuren insbesondere Ectoin ((S)-1,4,5,6-Tetrahydro-2-methyl-4-pyrimidincarbonsäure) und Hydroxyectoin ((S,S)-1,4,5,6-Tetrahydro-5-hydroxy-2-methyl-4-pyrimidincarbonsäure und deren Derivate zu nennen. Diese Verbindungen stabilisieren Enzyme und andere Biomoleküle in wässrigen Lösungen und organischen Lösungsmitteln. Weiter stabilisieren sie insbesondere Enzyme gegen denaturierende Bedingungen, wie Salze, extreme pH-Werte, Tenside, Harnstoff, Guanidiniumchlorid und andere Verbindungen.

Ectoin und Ectoin-Derivate wie Hydroxyectoin können vorteilhaft in Arzneimitteln verwendet werden. Insbesondere kann Hydroxyectoin zur Herstellung eines Arzneimittels zur Behandlung von Hauterkrankungen eingesetzt werden. Andere Einsatzgebiete des Hydroxyectoins und anderer Ectoin-Derivate liegen typischerweise in Gebieten in denen z.B. Trehalose als Zusatzstoff verwendet wird. So können Ectoin-Derivate, wie Hydroxyectoin, als Schutzstoff in getrockneten Hefe- und Bakterienzellen Verwendung finden. Auch pharmazeutische Produkte wie nicht glykosylierte, pharmazeutische wirksame Peptide und Proteine z.B. t-PA können mit Ectoin oder seinen Derivaten geschützt werden.

Unter den kosmetischen Anwendungen ist insbesondere die Verwendung von Ectoin und Ectoin-Derivaten zur Pflege von gealterter, trockener oder gereizter Haut zu nennen. So wird in der europäischen Patentanmeldung EP-A-0 671 161 insbesondere beschrieben, dass Ectoin und Hydroxyectoin in kosmetischen Zubereitungen wie Pudern, Seifen, tensidhaltigen Reinigungsprodukten, Lippenstiften, Rouge, Make-Ups, Pflegecremes und Sonnenschutzpräparaten eingesetzt werden.

Dabei wird vorzugsweise eine Pyrimidincarbonsäure gemäß der unten stehenden Formel eingesetzt,

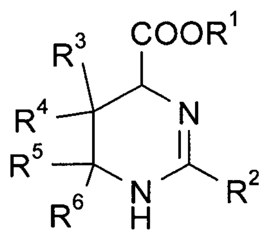

worin $R^1$ ein Rest H oder C1-8-Alkyl, $R^2$ ein Rest H oder C1-4-Alkyl und $R^3$, $R^4$, $R^5$ sowie $R^6$ jeweils unabhängig voneinander ein Rest aus der Gruppe H, OH, $NH_2$ und C1-4-Alkyl sind. Bevorzugt werden Pyrimidincarbonsäuren eingesetzt, bei denen $R^2$ eine Methyl- oder eine Ethylgruppe ist und $R^1$ bzw. $R^5$ und $R^6$ H sind. Insbesondere bevorzugt werden die Pyrimidincarbonsäuren Ectoin ((S)-1,4,5,6-Tetrahydro-2-methyl-4-pyrimidin-carbonsäure) und Hydroxyectoin ((S, S)-1,4,5,6-Tetrahydro-5-hydroxy-2-methyl-4-pyrimidin-carbonsäure) eingesetzt. Dabei enthalten die Zubereitungen derartige Pyrimidincarbonsäuren vorzugsweise in Mengen bis zu 15 Gew.-%. Vorzugsweise werden die Pyrimidincarbonsäuren dabei in Verhältnissen von 100:1 bis 1:100 zu den erfindungsgemäßen Verbindungen eingesetzt, wobei Verhältnisse im Bereich 1:10 bis 10:1 besonders bevorzugt sind.

Unter den Aryloximen wird vorzugsweise 2-Hydroxy-5-methyllaurophenonoxim, welches auch als HMLO, LPO oder F5 bezeichnet wird, eingesetzt. Seine Eignung zum Einsatz in kosmetischen Mitteln ist beispielsweise aus der Deutschen Offenlegungsschrift DE-A-41 16 123 bekannt. Zubereitungen, die 2-Hydroxy-5-methyllaurophenonoxim enthalten, sind demnach zur Behandlung von Hauterkrankungen, die mit Entzündungen einhergehen, geeignet. Es ist bekannt, dass derartige Zubereitungen z.B. zur Therapie der Psioriasis, unterschiedlicher Ekzemformen, irritativer und toxischer Dermatitis, UV-Dermatitis sowie weiterer allergischer und/oder entzündlicher Erkrankungen der Haut und der Hautanhangsgebilde verwendet werden können. Zubereitungen, die neben der erfindungsgemäßen Verbindung zusätzlich ein Aryloxim, vorzugsweise 2-Hydroxy-5-methyllaurophenonoxim enthalten, zeigen überraschende antiinflammatorische Eignung. Dabei enthalten die Zubereitungen vorzugsweise 0,01 bis 10 Gew.-% des Aryloxims, wobei es insbesondere bevorzugt ist, wenn die Zubereitung 0,05 bis 5 Gew-% Aryloxim enthält.

In einer weiteren Ausführungsform enthält die Zubereitung mindestens einen Selbstbräuner.

Als vorteilhafte Selbstbräuner können unter anderem Triosen und Tetrosen, wie beispielsweise die folgenden Verbindungen, eingesetzt werden:

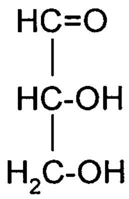

Glycerolaldehyd

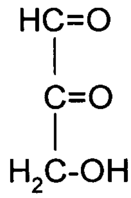

Hydroxymethylglyoxal

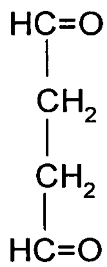

γ-Dialdehyd

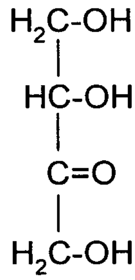

Erythrulose

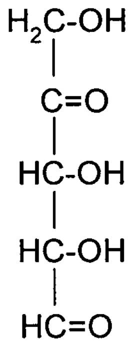

6-Aldo-D-Fructose

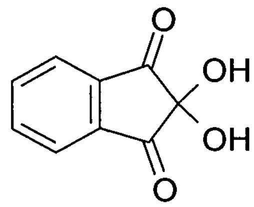

Ninhydrin

Ferner ist das 5-Hydroxy-1,4-naphtochinon (Juglon) zu nennen, das aus den Schalen frischer Walnüsse extrahiert werden kann sowie das in den Henna-Blättern vorkommende 2-Hydroxy-1,4-naphtochinon (Lawson). Auch das Flavonoid Diosmetin und seine Glycosides oder Sulfates können eingesetzt werden. Dabei können diese Verbindungen in Form von Reinstoffen oder Pflanzenextrakten eingesetzt werden. Diosmetin kann beispielsweise vorzzugsweise in Form eines Chrysanthemum Extraktes eingesetzt werden.

Ganz besonders bevorzugt ist das 1,3-Dihydroxyaceton (DHA), ein im menschlichen Körper vorkommender dreiwertiger Zucker und dessen Derivate.

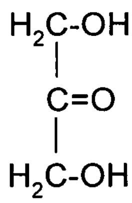

1,3-Dihydroxyaceton (DHA)

Dabei können die genannten Selbstbräuner alleine oder als Gemisch eingesetzt werden. Insbesondere bevorzugt ist es dabei, wenn DHA im Gemisch mit einem weiteren der oben genannten Selbstbräuner eingesetzt wird.

Alle Verbindungen oder Komponenten, die in den Zubereitungen verwendet werden können, sind entweder bekannt und käuflich erwerbbar oder können nach bekannten Verfahren synthetisiert werden.

Die erfindungsgemäße Verbindung kann in der üblichen Weise in kosmetische oder dermatologische Zubereitungen eingearbeitet werden. Geeignet sind Zubereitungen für eine äußerliche Anwendung, beispielsweise als Creme, Lotion, Gel, oder als Lösung, die auf die Haut aufgesprüht werden kann. Für eine innerliche Anwendung sind Darreichungsformeln wie Kapseln, Dragees, Pulver, Tabletten-Lösungen oder Lösungen geeignet.

Als Anwendungsform der Zubereitungen seien z.B. genannt: Lösungen, Suspensionen, Emulsionen, PIT-Emulsionen, Pasten, Salben, Gele, Cremes, Lotionen, Puder, Seifen, tensidhaltige Reinigungspräparate, Öle, Aerosole und Sprays. Weitere Anwendungsformen sind z.B. Sticks, Shampoos und Duschbäder. Der Zubereitung können beliebige übliche Trägerstoffe, Hilfsstoffe und gegebenenfalls weitere Wirkstoffe zugesetzt werden.

Vorzuziehende Hilfsstoffe stammen aus der Gruppe der Konservierungsstoffe, Antioxidantien, Stabilisatoren,

Lösungsvermittler, Vitamine, Färbemittel, Geruchsverbesserer.

Salben, Pasten, Cremes und Gele können die üblichen Trägerstoffe enthalten, z.B. tierische und pflanzliche Fette, Wachse, Paraffine, Stärke, Traganth, Cellulosederivate, Polyethylenglykole, Silicone, Bentonite, Kieselsäure, Talkum und Zinkoxid oder Gemische dieser Stoffe.

Puder und Sprays können die üblichen Trägerstoffe enthalten, z.B. Milchzucker, Talkum, Kieselsäure, Aluminiumhydroxid, Calciumsilikat und Polyamid-Pulver oder Gemische dieser Stoffe. Sprays können zusätzlich die üblichen Treibmittel, z.B. Chlorfluorkohlenwasserstoffe, Propan/Butan oder Dimethylether, enthalten.

Lösungen und Emulsionen können die üblichen Trägerstoffe wie Lösungsmittel, Lösungsvermittler und Emulgatoren, z.B. Wasser, Ethanol, Isopropanol, Ethylcarbonat, Ethlyacetat, Benzylalkohol, Benzylbenzoat, Propylenglykol, 1,3-Butylglykol, Öle, insbesondere Baumwollsaatöl, Erdnussöl, Maiskeimöl, Olivenöl, Rizinusöl und Sesamöl, Glycerinfettsäureester, Polyethylenglykole und Fettsäureester des Sorbitans oder Gemische dieser Stoffe enthalten.

Suspensionen können die üblichen Trägerstoffe wie flüssige Verdünnungsmittel, z.B. Wasser, Ethanol oder Propylenglykol, Suspendiermittel, z.B. ethoxylierte Isostearylalkohole, Polyoxyethylensorbitester und Polyoxyethylensorbitanester, mikrokristalline Cellulose, Aluminiummetahydroxid, Bentonit, Agar-Agar und Traganth oder Gemische dieser Stoffe enthalten.

Seifen können die üblichen Trägerstoffe wie Alkalisalze von Fettsäuren, Salze von Fettsäurehalbestern, Fettsäureeiweißhydrolysaten, Isothionate, Lanolin, Fettalkohol, Pflanzenöle, Pflanzenextrakte, Glycerin, Zucker oder Gemische dieser Stoffe enthalten.

Tensidhaltige Reinigungsprodukte können die üblichen Trägerstoffe wie Salze von Fettalkoholsulfaten, Fettalkoholethersulfaten, Sulfobernsteinsäurehalbestern, Fettsäureeiweißhydrolysaten, Isothionate, Imidazoliniumderivate, Methyltaurate, Sarkosinate, Fettsäureamidethersulfate, Alkylamidobetaine, Fettalkohole, Fettsäureglyceride, Fettsäurediethanolamide, pflanzliche und synthetische Öle, Lanolinderivate, ethoxylierte Glycerinfettsäureester oder Gemische dieser Stoffe enthalten.

Gesichts- und Körperöle können die üblichen Trägerstoffe wie synthetische Öle wie Fettsäureester, Fettalkohole, Silikonöle, natürliche Öle wie Pflanzenöle und ölige Pflanzenauszüge, Paraffinöle, Lanolinöle oder Gemische dieser Stoffe enthalten.

Weitere typische kosmetische Anwendungsformen sind auch Lippenstifte, Lippenpflegestifte, Mascara, Eyeliner, Lidschatten, Rouge, Puder-, Emulsions- und Wachs-Make up sowie Sonnenschutz-, Prä-Sun- und After-Sun-Präparate.

Zu den bevorzugten Zubereitungsformen gehören insbesondere Emulsionen.

Emulsionen sind vorteilhaft und enthalten z. B. die genannten Fette, Öle, Wachse und anderen Fettkörper, sowie Wasser und einen Emulgator, wie er üblicherweise für einen solchen Typ der Zubereitung verwendet wird.

Die Lipidphase kann vorteilhaft gewählt werden aus folgender Substanzgruppe:

- Mineralöle, Mineralwachse
- Öle, wie Triglyceride der Caprin- oder der Caprylsäure, ferner natürliche Öle wie z. B. Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fett-Ikoholen mit Alkansäuren niedriger C-Zahl oder mit Fettsäuren;
- Silikonöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigtem und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäure und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexaldecylstearat, 2-Octyldodecylpalmitat, Oleyloleat, Oleylerucat, Erucyloleat, Erucylerucat sowie synthetische, halbsynthetische und natürliche Gemische solcher Ester, z. B. Jojobaöl.

Ferner kann die Ölphase vorteilhaft gewählt werden aus der Gruppe der verzweigten und unverzweigten Kohlenwasserstoffe und -wachse, der Silikonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12-18 C-Atomen. Die Fettsäuretriglyceride können beispielsweise vorteilhaft gewählt werden aus der Gruppe der synthetischen, halbsynthetischen und natürlichen Öle, z. B. Olivenöl, Sonnenblumenöl, Sojaöl, Erdnussöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, $C_{12}$-$_{15}$-Alkylbenzoat, Capryl-Caprinsäure-triglycerid, Dicaprylether.

Besonders vorteilhaft sind Mischungen aus $C_{12}$-$_{15}$-Alkylbenzoat und 2-Ethylhexylisostearat, Mischungen aus $C_{12}$-$_{15}$-Alkylbenzoat und Isotridecylisononanoat sowie Mischungen aus $C_{12}$-$_{15}$-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann auch die Ölphase ferner einen Gehalt an cyclischen oder linearan Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als zu verwendendes Silikonöl eingesetzt. Aber auch andere Silikonöle sind vorteilhaft zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die wässrige Phase der Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z. B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Insbesondere werden Gemische der vorstehend genannten Lösemittel verwendet. Bei alkoholischen Lösemitteln kann Wasser ein weiterer Bestandteil sein.

Emulsionen sind vorteilhaft und enthalten z. B. die genannten Fette, Öle, Wachse und anderen Fettkörper, sowie Wasser und einen Emulgator, wie er üblicherweise für einen solchen Typ der Formuierung verwendet wird.

In einer bevorzugten Ausführungsform enthalten die Zubereitungen hydrophile Tenside.

Die hydrophilen Tenside werden bevorzugt gewählt aus der Gruppe der Alkylglucoside, der Acyllactylate, der Betaine sowie der Cocoamphoacetate.

Die Alkylglucoside werden ihrerseits vorteilhaft gewählt aus der Gruppe der Alkylglucoside, welche sich durch die Strukturformel

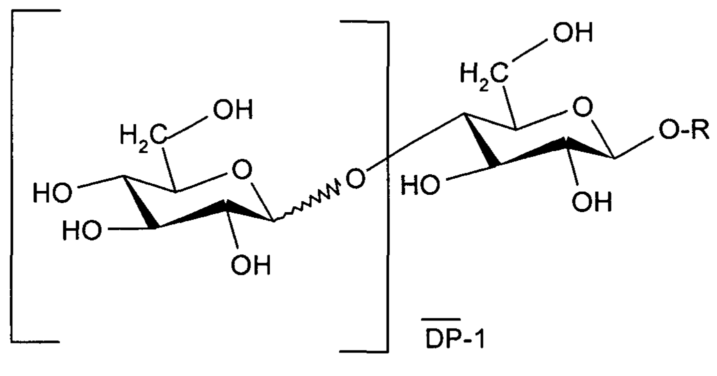

auszeichnen, wobei R einen verzweigten oder unverzweigten Alkylrest mit 4 bis 24 Kohlenstoffatomen darstellt und wobei DP einen mittleren Glucosylierungsgrad von bis zu 2 bedeutet.

Der Wert DP repräsentiert den Glucosidierungsgrad der erfindungsgemäß verwendeten Alkylglucoside und ist definiert als

$$\overline{DP} = \frac{p_1}{100} \cdot 1 + \frac{p_2}{100} \cdot 2 + \frac{p_3}{100} \cdot 3 + \ldots = \sum \frac{p_i}{100} \cdot i$$

Dabei stellen $p_1$, $p_2$, $p_3$ ... bzw. $p_i$ den Anteil der einfach, zweifach dreifach ... i-fach glucosylierten Produkte in Gewichtsprozenten dar. Vorteilhaft werden Produkte mit Glucosylierungsgraden von 1-2, insbesondere vorteilhaft von 1, 1 bis 1,5, ganz besonders vorteilhaft von 1,2-1,4, insbesondere von 1,3 gewählt.

Der Wert DP trägt den Umstande Rechnung, dass Alkylglucoside herstellungsbedingt in der Regel Gemische aus Mono- und Oligoglucosiden darstellen. Vorteilhaft ist ein relativ hoher Gehalt an Monoglucosiden, typischerweise in der Größenordnung von 40-70 Gew.-%.

Vorteilhaft verwendete Alkylglylcoside werden gewählt aus der Gruppe Octylglucopyranosid, Nonylglucopyranosid, Decylglucopyranosid, Undecylglucopyranosid, Dodecylglucopyranosid, Tetradecylglucopyranosid und Hexadecylglucopyranosid.

Es ist ebenfalls von Vorteil, natürliche oder synthetische Roh- und Hilfsstoffe bzw. Gemische einzusetzen, beispielsweise Plantaren® 1200 (Henkel KGaA), Oramix® NS 10 (Seppic).

Die Acyllactylate werden ihrerseits vorteilhaft gewählt aus der Gruppe der Substanzen, welche sich durch die Strukturformel

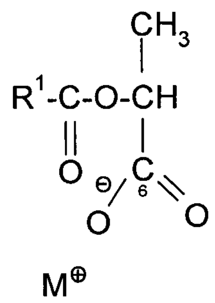

auszeichnen, wobei $R^1$ einen verzweigten oder unverzweigten Alkylrest mit 1 bis 30 Kohlenstoffatomen bedeutet und $M^+$ aus der Gruppe der Alkaliionen sowie der Gruppe der mit einer oder mehreren Alkyl- und/oder mit einer oder mehreren Hydroxyalkylresten substituierten Ammoniumionen gewählt wird bzw. dem halben Äquivalent eines Erdalkalions entspricht.

Vorteilhaft ist beispielsweise Natriumisostearyllactylat, beispielsweise das Produkt Pathionic® ISL von der Gesellschaft American Ingredients Company.

Die Betaine werden vorteilhaft gewählt aus der Gruppe der Substanzen, welche sich durch die Strukturformel

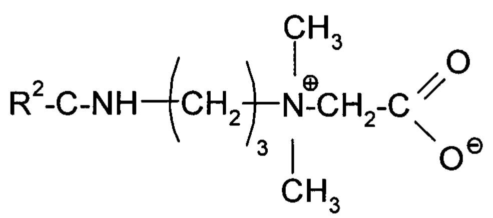

auszeichnen, wobei $R^2$ einen verzweigten oder unverzeigten Alkylrest mit 1 bis 30 Kohlenstoffatomen bedeutet.

Insbesondere vorteilhaft bedeutet $R^2$ einen verzweigten oder unverzweigten Alkylrest mit 6 bis 12 Kohlenstoffatomen.

Vorteilhaft ist beispielsweise Capramidopropylbetain, beispielsweise das Produkt Tego® Betain 810 von der Gesellschaft Th. Goldschmidt AG.

Als vorteilhaftes Cocoamphoacetat wird beispielsweise Natriumcocoamphoacetat gewählt, wie es unter der Bezeichnung Miranol® Ultra C32 von der Gesellschaft Miranol Chemical Corp. erhältlich ist.

Die Zubereitungen sind vorteilhaft dadurch gekennzeichnet, dass das oder die hydrophilen Tenside in Konzentrationen von 0,01-20 Gew.-% bevorzugt 0,05-10 Gew.-%, besonders bevorzugt 0,1-5 Gew.-%, jeweils bezogen auf das Gesamtgewicht der Zusammensetzung, vorliegt oder vorliegen.

Zu Anwendung werden die kosmetischen und dermatologischen Zubereitungen in der für Kosmetika üblichen Weise auf die Haut und/oder die Haare in ausreichender Menge aufgebracht.

Kosmetische und dermatologische Zubereitungen können in verschiedenen Formen vorliegen. So können sie z. B. eine Lösung, eine wasserfreie Zubereitung, eine Emulsion oder Mikroemulsion vom Typ Wasser-in-Öl (W/O) oder vom Typ Öl-in-Wasser (O/W), eine multiple Emulsion, beispielsweise vom Typ Waser-in-Öl-in-Wasser (W/O/W), ein Gel, einen festen Stift, eine Salbe oder auch ein Aerosol darstellen. Es ist auch vorteilhaft, Wirkstoffe in verkapselter Form darzureichen, z. B. in Kollagenmatrices und anderen üblichen Verkapselungsmaterialien, z. B. als Celluloseverkapselungen, in Gelatine, Wachsmatrices oder liposomal verkapselt. Insbesondere Wachsmatrices wie sie in der DE-OS 43 08 282 beschrieben werden, haben sich als günstig herausgestellt. Bevorzugt werden Emulsionen. O/W-Emulsionen werden besonders bevorzugt. Emulsionen, W/O-Emulsionen und O/W-Emulsionen sind in üblicher Weise erhältlich.

Als Emulgatoren können beispielsweise die bekannten W/O- und O/W-Emulgatoren verwendet werden. Es ist vorteilhaft, weitere übliche Co-emulgatoren in den erfindungsgemäßen bevorzugten O/W-Emulsionen zu verwenden.

Vorteilhaft werden als Co-Emulgatoren beispielsweise O/W-Emulgatoren gewählt, vornehmlich aus der Gruppe der Substanzen mit HLB-Werten von 11-16, ganz besonders vorteilhaft mit HLB-Werten von 14,5-15,5, sofern die O/W-Emulgatoren gesättigte Reste R und R' aufweisen. Weisen die O/W-Emulgatoren ungesättigte Reste R und/oder R' auf, oder liegen Isoalkylderivate vor, so kann der bevorzugte HLB-Wert solcher Emulgatoren auch niedriger oder darüber liegen.

Es ist von Vorteil, die Fettalkoholethoxylate aus der Gruppe der ethoxylierten Stearylalkhole, Cetylalkohole, Cetylstearylalkohole (Cetearylalkohole) zu wählen. Insbesondere bevorzugt sind: Polyethylenglycol(13)stearylether (Steareth-13), Polyethylenglycol(14)stearylether (Steareth-14), Polyethylenglycol(15)stearylether (Steareth-15), Polyethylenglycol(16)stearylether (Steareth-16), Polyethylenglycol(17)-stearylether (Steareth-17),Polyethylenglycol(18)stearylether (Steareth-18), Polyethylenglycol(19)stearylether (Steareth-19), Polyethylenglycol(20)-stearylether (Steareth-20), Polyethylenglycol(12)isostearylether (Isosteareth-12), Polyethylenglycol(13)isostearylether (Isosteareth-13), Polyethylenglycol(14)isostearylether (Isosteareth-14), Polyethylenglycol(15)isostearylether (Isosteareth-15), Polyethylenglycol(16)-isostearylether (Isosteareth-16), Polyethylenglycol(17)isostearylether (Isosteareth-17), Polyethylenglycol(18)isostearylether (Isosteareth-18), Polyethylenglycol(19)isostearylether (Isosteareth-19), Polyethylenglycol(20)isostearylether (Isosteareth-20), Polyethylenglycol(13)cetylether (Ceteth-13), Polyethytenglycol(14)cetylether (Ceteth-14), Polyethylenglycol(15)cetylether (Ceteth-15), Polyethylenglycol(16)cetylether (Ceteth-16), Polyethylenglycol(17)cetylether (Ceteth-17), Polyethylenglycol(18)-cetylether (Ceteth-18), Polyethylenglycol(19)cetylether (Ceteth-19), Polyethylen-glycol(20)cetylether (Ceteth-20), Polyethylenglycol(13)isocetylether (Isoceteth-13), Polyethylenglycol(14)isocetylether (Isoceteth-14), Polyethylenglycol(15)isocetylether (Isoceteth-15), Polyethylenglycol(16)isocetylether (Isoceteth-16), Polyethylenglycol(17)-isocetylether (Isoceteth-17), Polyethylenglycol(18)isocetylether (Isoceteth-18), Polyethylenglycol(19)isocetylether (Isoceteth-19), Polyethylenglycol(20)isocetylether (Isoceteth-20), Polyethylenglycol(12)oleylether (Oleth-12), Polyethylenglycol(13)oleylether (Oleth-13), Polyethylenglycol(14)oleylether (Oleth-14), Polyethylenglycol(15)oleylether (Oleth-15), Polyethylenglycol(12)laurylether (Laureth-12), Polyethylenglycol(12)-isolaurylether (Isolaureth-12), Polyethylenglycol(13)cetylstearylether (Ceteareth-13), Polyethylenglycol(14)cetylstearylether (Ceteareth-14), Polyethylenglycol(15)cetylstearylether (Ceteareth-15), Polyethylenglycol(16)cetylstearylether (Ceteareth-16), Polyethylenglycol(17)-cetylstearylether (Ceteareth-17), Polyethylenglycol(18)cetylstearylether (Ceteareth-18), Polyethylenglycol(19)cetylstearylether (Ceteareth-19), Polyethylenglycol(20)cetylstearylether (Ceteareth-20).

Es ist ferner von Vorteil, die Fettsäureethoxylate aus folgender Gruppe zu wählen: Polyethylenglycol(20)stearat, Polyethylenglycol(21)stearat, Polyethylenglycol(22)stearat, Polyethylenglycol(23)stearat, Polyethylenglycol(24)stearat, Polyethylenglycol(25)stearat, Polyethylenglycol(12)isostearat, Polyethylenglycol(13)isostearat, Polyethylenglycol(14)isostearat, Polyethylenglycol(15)isostearat, Polyethylenglycol(16)isostearat, Polyethylenglycol(17)isostearat, Polyethylenglycol(18)isostearat, Polyethylenglycol(19)isostearat, Polyethylenglycol(20)isostearat, Polyethylenglycol(21)isostearat, Polyethylenglycol(22)isostearat, Polyethylenglycol(23)isostearat, Polyethylenglycol(24)isostearat, Polyethylenglycol(25)isostearat, Polyethylenglycol(12)oleat, Polyethylenglycol(13)oleat, Polyethylenglycol(14)oleat, Polyethylenglycol(15)oleat, Polyethylenglycol(16)oleat, Polyethylenglycol(17)oleat, Polyethylenglycol(18)oleat, Polyethylenglycol(19)oleat, Polyethylenglycol(20)oleat

Als ethoxylierte Alkylethercarbonsäure bzw. deren Salz kann vorteilhaft das Natriumlaureth-11-carboxylat verwendet werden. Als Alkylethersulfat kann Natrium Laureth1-4sulfat vorteilhaft verwendet werden. Als ethoxyliertes Cholesterinderivat kann vorteilhaft Polyethylenglycol(30)Cholesterylether verwendet werden. Auch Polyethylenglycol(25)Sojasterol hat sich bewährt. Als ethoxylierte Triglyceride können vorteilhaft die Polyethylenglycol(60) Evening Primrose Glycerides verwendet werden (Evening Primrose = Nachtkerze).

Weiterhin ist von Vorteil, die Polyethylenglycolglycerinfettsäureester aus der Gruppe Polyethylenglycol(20)glyceryllaurat, Polyethylenglycol(21)glyceryllaurat, Polyethylenglycol(22)glyceryllaurat, Polyethylenglycol(23)glyceryllaurat, Polyethylenglycol(6)glycerylcaprat/cprinat, Polyethylenglycol-(20)glyceryloleat, Polyethylenglycol(20)glycerylisostearat, Polyethylenglycol(18)glyceryloleat(cocoat zu wählen.

Es ist ebenfalls günstig, die Sorbitanester aus der Gruppe Polyethylenglycol(20)sorbitanmonolaurat, Polyethylenglycol(20)sorbitanmonostearat, Polyethylenglycol(20)sorbitanmonoisostearat, Polyethylenglycol(20)sorbitanmonopalmitat, Polyethylenglycol(20)sorbitanmonooleat zu wählen.

Als fakultative, dennoch gegebenenfalls vorteilhafte W/O-Emulgatoren können eingesetzt werden:

Fettalkohole mit 8 bis 30 Kohlenstoffatomen, Monoglycerinester gesättigter und/oder ungesättigter, verzweigter und/oder unverzweigter Alkancarbonsäuren einer Kettenlänge von 8 bis 24, insbesondere 12-18 C-Atome, Diglycerinester gesättigter und/oder ungesättigter, verzweigter und/oder unverzweigter Alkancarbonsäuren einer Kettenlänge von 8 bis 24, insbesondere 12-18 C-Atomen, Monoglycerinether gesättigter und/oder ungesättigter, verzweigter und/oder unverzweigter Alkohole einer Kettenlänge von 8 bis 24, insbesondere 12-18 C-Atomen, Diglycerinether gesättigter und/oder ungesättigter, verzweigter und/oder unverzweigter Alkhole einer Kettenlänge von 8 bis 24, insbesondere 12-18 C-Atomen, Propylenglycolester gesättigter und/oder ungesättigter, verzweigter und/oder unverzweigter Alkancarbonsäuren einer Kettenlänge von 8 bis 24, insbesondere 12-18 C-Atomen sowie Sorbitanester gesättigter und/oder ungesättigter, verzweigter und/oder unverzweigter Alkancarbonsäuren einer Kettenlänge von 8 bis 24, insbesondere 12-18 C-Atomen.

Insbesondere vorteilhafte W/O-Emulgatoren sind Glycerylmonostearat, Glycerylmonoisostearat, Glycerylmonomyristat, Glycerylmonooleat, Diglycerylmonostearat, Diglycerylmonoisostearat, Propylenglycolmonostearat, Propylenglycolmonoisostearat, Propylenglycolmonocaprylat, Propylenglycolmonolaurat, Sorbitanmonoisostearat, Sorbitanmonolaurat, Sorbitanmonocaprylat, Sorbitanmonoisooleat, Saccharosedistearat, Cetylalkohol, Stearylalkohol, Arachidylalkohol, Behenylalkohol, Isobehenylalkohol, Selachylalkohol, Chimylalkohol, Polyethylenglycol(2)stearylether (Steareth-2), Glycerylmonolaurat, Glycerylmonocaprinat, Glycerylmonocaprylat.

Bevorzugte Zubereitungen eignen sich besonders zum Schutz menschlicher Haut gegen Alterungsprozesse sowie vor oxidativem Stress, d.h. gegen Schädigungen durch Radikale, wie sie z.B. durch Sonneneinstrahlung, Wärme oder andere Einflüsse erzeugt werden. Dabei liegt sie in verschiedenen, für diese Anwendung üblicherweise verwendeten Darreichungsformen vor. So kann sie insbesondere als Lotion oder Emulsion, wie als Creme oder Milch (O/W, W/O, O/W/O, W/O/W), in Form ölig-alkoholischer, ölig-wässriger oder wässrigalkoholischer Gele bzw. Lösungen, als feste Stifte vorliegen oder als Aerosol konfektioniert sein.

Die Zubereitung kann kosmetische Adjuvantien enthalten, welche in dieser Art von Zubereitungen üblicherweise verwendet werden, wie z.B. Verdickungsmittel, weichmachende Mittel, Befeuchtungsmittel, grenzflächenaktive Mittel, Emulgatoren, Konservierungsmittel, Mittel gegen Schaumbildung, Parfums, Wachse, Lanolin, Treibmittel, Farbstoffe und/oder Pigmente, welche das Mittel selbst oder die Haut färben, und andere in der Kosmetik gewöhnlich verwendete Ingredienzien.

Man kann als Dispersions- bzw. Solubilisierungsmittel ein Öl, Wachs oder sonstigen Fettkörper, einen niedrigen Monoalkohol oder ein niedriges Polyol oder Mischungen davon verwenden. Zu den besonders bevorzugten Monoalkoholen oder Polyolen zählen Ethanol, i-Propanol, Propylenglykol, Glycerin und Sorbit.

Eine bevorzugte Ausführungsform der Zubereitung ist eine Emulsion, welche als Schutzcreme oder -milch vorliegt und außer der oder den erfindungsgemäßen Verbindungen beispielsweise Fettalkohole, Fettsäuren, Fettsäureester, insbesondere Triglyceride von Fettsäuren, Lanolin, natürliche und synthetische Öle oder Wachse und Emulgatoren in Anwesenheit von Wasser enthält.

Weitere bevorzugte Ausführungsformen stellen ölige Lotionen auf Basis von natürlichen oder synthetischen Ölen und Wachsen, Lanolin, Fettsäureestern, insbesondere Triglyceriden von Fettsäuren, oder öligalkoholische Lotionen auf Basis eines Niedrigalkohols, wie Ethanol, oder eines Glycerols, wie Propylenglykol, und/oder eines Polyols, wie Glycerin, und Ölen, Wachsen und Fettsäureestern, wie Triglyceriden von Fettsäuren, dar.

Die Zubereitung kann auch als alkoholisches Gel vorliegen, welches einen oder mehrere Niedrigalkohole oder -polyole, wie Ethanol, Propylenglykol oder Glycerin, und ein Verdickungsmittel, wie Kieselerde umfaßt. Die öligalkoholischen Gele enthalten außerdem natürliches oder synthetisches Öl oder Wachs.

Die festen Stifte bestehen aus natürlichen oder synthetischen Wachsen und Ölen, Fettalkoholen, Fettsäuren, Fettsäureestern, Lanolin und anderen Fettkörpern.

Ist eine Zubereitung als Aerosol konfektioniert, verwendet man in der Regel die üblichen Treibmittel, wie Alkane, Fluoralkane und Chlorfluoralkane.

Die kosmetische Zubereitung kann auch zum Schutz der Haare gegen fotochemische Schäden verwendet werden, um Veränderungen von Farbnuancen, ein Entfärben oder Schäden mechanischer Art zu verhindern. In diesem Fall erfolgt geeignet eine Konfektionierung als Shampoo, Lotion, Gel oder Emulsion zum Ausspülen, wobei die jeweilige Zubereitung vor oder nach dem Shamponieren, vor oder nach dem Färben oder Entfärben bzw. vor oder nach der Dauerwelle aufgetragen wird. Es kann auch eine Zubereitung als Lotion oder Gel zum Frisieren und Behandeln, als Lotion oder Gel zum Bürsten oder Legen einer Wasserwelle, als Haarlack, Dauerwellenmittel, Färbe- oder Entfärbemittel der Haare gewählt werden. Die Zubereitung mit Lichtschutzeigenschaften kann außer der oder den erfindungsgemäßen Verbindungen verschiedene, in diesem Mitteltyp verwendete Adjuvantien enthalten, wie Grenzflächen aktive Mittel, Verdickungsmittel, Polymere, weichmachende Mittel, Konservierungsmittel, Schaumstabilisatoren, Elektrolyte, organische Lösungsmittel, Silikonderivate, Öle, Wachse, Antifettmittel, Farbstoffe und/oder Pigmente, die das Mittel selbst oder die Haare färben oder andere für die Haarpflege üblicherweise verwendete Ingredienzien.

Die Zubereitungen können dabei mit Hilfe von Techniken hergestellt werden, die dem Fachmann wohl bekannt sind.

Das Vermischen kann ein Lösen, Emulgieren oder Dispergieren der erfindungsgemäßen Verbindung in dem Träger zur Folge haben.

In einem erfindungsgemäß bevorzugten Verfahren wird die Verbindung 4-Hydroxy-3,5-dimethoxy-benzyl-malonsäure-di-2-ethylhexylester hergestellt durch Hydrierung.

Zur Hydrierung ist z. B. molekularer Wasserstoff geeignet. Wenn molekularer Wasserstoff für die Hydrierung verwendet wird, geschieht die Hydrierung vorzugsweise in Gegenwart eines Katalysators bzw. Katalysatorsystems.

Als Katalysatoren für die Hydrierung eignen sich alle gängigen homogenen und heterogenen Katalysatoren, insbesondere bevorzugt wird als Katalysator mindestens ein Edelmetall vorzugsweise ausgewählt aus den Elementen Pt, Pd und Rh, oder ein Übergangsmetall, wie Mo, W, Cr, besonders aber Fe, Co und Ni, entweder einzeln oder im Gemisch eingesetzt. Dabei können der oder die Katalysatoren oder Katalysatorgemische auch auf Trägern wie Kohle, Aktivkohle, Aluminiumoxid, Bariumcarbonat, Bariumsulfat, Calciumcarbonat, Strontiumcarbonat oder Kieselgur eingesetzt werden. Dabei kann das Metall auch in Form der Raney-Verbindung, beispielsweise Raney-Nickel eingesetzt werden. Wird die Katalyse in einem homogenen Verfahren durchgeführt, so ist es bevorzugt, wenn als Katalysator eine oder mehrere Komplexverbindungen der genannten Metalle, wie beispielsweise der Wilkinson-Katalysator [Chlor-tris(triphenylphosphin)rhodium], eingesetzt wird. Es können ferner Salze der genannten Metalle eingesetzt werden, die in situ durch ein Reduktionsmittel reduziert werden können und in situ eine fein verteilte Metall (0)-Spezies erzeugen. Geeignete Edelmetallsalze sind beispielsweise Palladiumacetat, Palladiumbromid oder Palladiumchlorid, geeignete Reduktionsmittel sind beispielsweise Wasserstoff, Hydrazin, Natriumborhydrid oder Formiate. In einer bevorzugten Variante der vorliegenden Erfindung wir ein heterogener Katalysator eingesetzt, wobei es insbesondere bevorzugt ist als Katalysator bei dem erfindungsgemäßen Verfahren Pd oder Pt, vorzugsweise auf Aktivkohleträger, beispielsweise 5 Gew.-% Pd oder Pt auf C, einzusetzen.

Die Hydrierung wird üblicherweise bei einer Temperatur im Bereich von 20 - 150°C durchgeführt. Weiter wird die Hydrierung vorteilhaft bei einem Wasserstoff-Druck von 1 bis 200 bar durchgeführt.

Als Lösungsmittel eignen sich protische Lösungsmittel, insbesondere die dem Fachmann bekannten üblichen protischen Lösungsmittel, wie Wasser, niedere Alkohole, wie beispielsweise Methanol, Ethanol und Isopropanol, sowie primäre und sekundäre Amine und Gemische solcher protischer Lösungsmittel, wobei es insbesondere bevorzugt sein kann, wenn als Lösungsmittel Wasser eingesetzt wird.

Als Lösungsmittel für diese Umsetzung eignen sich weiter auch übliche aprotische Lösungsmittel. Beispielsweise können Diethylether, Tetrahydrofuran, Benzol, Toluol, Acetonitril, Dimethoxyethan, Dimethylformamid, Dimethylsulfoxid und N-Methyl-pyrrolidon eingesetzt werden.

In einer ebenfalls bevorzugten Ausführungsform des erfindungsgemäßen Herstellverfahrens wird die Hydrierung in Substanz durchgeführt, d.h. es ist kein zusätzliches Lösungsmittel erforderlich.

Nach beendeter Reaktion kann die Aufarbeitung nach üblichen Methoden erfolgen. Beispielsweise kann der Katalysator abfiltriert, das Filtrat vom Lösungsmittel befreit werden, z. B. durch Erhitzen bei im Vergleich zu Atmosphärendruck erniedrigtem Druck und das so erhaltene Produkt durch übliche Methoden weiter aufgereinigt werden.

Die weitere Aufreinigung der Reaktionsprodukte kann ebenfalls durch übliche Methoden, beispielsweise durch Umkristallisieren aus einem geeigneten Lösungsmittel, oder durch chromatographische Methoden erfolgen.

Es wurde auch festgestellt, dass die erfindungsgemäße Verbindung stabilisierend auf die Zubereitung wirken kann. Bei der Verwendung in entsprechenden Produkten bleiben diese daher auch länger stabil und verändern ihre galenische und sensorische Beschaffenheit nicht.

Insbesondere bleibt auch bei längerdauernder Anwendung bzw. längerer Lagerung die Wirksamkeit der Inhaltsstoffe, z.B. Vitamine, erhalten. Dies ist unter anderem besonders vorteilhaft bei Zusammensetzungen zum Schutz der Haut gegen die Einwirkung von UV-Strahlen, da diese Kosmetika besonders hohen Belastungen durch die UV-Strahlung ausgesetzt sind.

Die positiven Wirkungen der erfindungsgemäßen Verbindung ergibt deren besondere Eignung zur Verwendung in kosmetischen oder pharmazeutischen Zubereitungen.

Durch ihre Wirkung als Antioxidationsmittel bzw. als Radikalfänger eignet sich die erfindungsgemäße Verbindung auch als Arzneimittelinhaltsstoff. Sie wirken dabei unterstützend oder substituierend zu natürlichen Mechanismen, welche Radikale im Körper abfangen. Die erfindungsgemäße Verbindung kann in ihrer Wirkung teilweise mit Radikalfängern wie Vitamin C verglichen werden. Die erfindungsgemäße Verbindung kann beispielsweise zur vorbeugenden Behandlungen von Entzündungen und Allergien der Haut sowie in bestimmten Fällen zur Verhütung bestimmter Krebsarten verwendet werden. Insbesondere eignet sich die erfindungsgemäße Verbindung zur Herstellung eines Arzneimittels zur Behandlung von Entzündungen, Allergien und Irritationen, insbesondere der Haut. Ferner können Arzneimittel hergestellt werden in einer Wirkung als Venentonikum, als Mittel zur Erhöhung der Festigkeit von Blutkapillaren, als Hemmstoff für Cuperose, als Hemmstoff chemischer, physikalischer oder aktinischer Erytheme, als Mittel zur Behandlung empfindlicher Haut, als Dekongestionsmittel, als Entwässerungsmittel, als Mittel zum Schlankmachen, als Antifaltenmittel, als Stimulatoren der Synthese von Komponenten der extrazellulären Matrix, als stärkendes Mittel zur Verbesserung der Hautelastizität und als Antialterungsmittel. Weiter zeigt in diesem Zusammenhang die erfindungsgemäße Verbindung antiallergische und antiinflammatorische und antiirritative Wirkungen. Sie eignet sich daher zur Herstellung von Arzneimitteln zur Behandlung von Entzündungen oder allergischen Reaktionen.

Im folgenden wird die Erfindung anhand von Beispielen näher erläutert.

**Beispiele**

**Beispiel 1: Herstellung von 4-Hydroxy-3,5-dimethoxy-benzylmalonsäure-di 2-ethyl-hexylester**

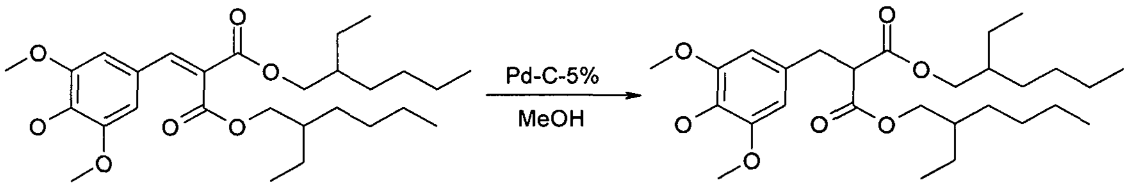


(4-Hydroxy-3,5-dimethoxy-benzyliden)-malonsäure-di-2-ethyl-hexylester (Die Synthese dieser Verbindung ist in WO-A-2003/007906 beschrieben) wird in Methanol (14 ml/mmol) gelöst und Pd-C-5% (56% Wasser; Merck: Art.-No. 275175; 0,54 g/mmol) zugegeben. Anschließend erfolgt die Hydrierung mit Wasserstoff 3.0 bei Raumtemperatur und Normaldruck. Der Katalysator wird durch Filtration abgetrennt. Das Filtrat wird im Vakuum vom Lösungsmittel befreit und das rückbleibende grünliche Öl in tert-Butylmethylether (MTBE) aufgenommen und 2 x mit 1 N HCl, 1 x gesättigter, wässriger NaHCO3-Lösung und 1 x gesättigter, wässriger NaCl-Lösung extrahiert. Die organische Phase wird über Natriumsulfat getrocknet und das Lösungsmittel im Vakuum entfernt. Die Reinigung erfolgt durch Filtration über Kieselgel. Hierzu wird das Rohprodukt in Petrolether (PE) aufgenommen und mit PE/MTBE eluiert. Man erhält analysenreines Produkt als farbloses Öl.

**Beispiel 2: Oxidation im UV Licht**

Figur 1 zeigt die Veränderung des UV-Spektrums von 4-Hydroxy-3,5-dimethoxy-benzyl-malonsäure-di 2-ethylhexylester (aus Beispiel 1) bei Bestrahlung mit UV-Licht.

Die Kurven stehen für die unbestrahlte Substanz (0 kJ/$m^2$ eingestrahlt), nach 15 min Bestrahlung (86 kJ/$m^2$ eingestrahlt), nach 65 min Bestrahlung (373 kJ/$m^2$ eingestrahlt), nach 235 min Bestrahlung (1349 kJ/$m^2$ eingestrahlt) und nach 405 min Bestrahlung (2325 kJ/$m^2$ eingestrahlt). Die Spektren sind auf einem Carry 300 bio Spektrometer aufgenommen. Die Bestrahlung erfolgt mittels einer Atlas Sun Test CPS, Xenon Lamp mit UV-Spezialglas-Filter bei einer Leistung von 95,69 W/$m^2$ im Bereich 290 - 400 nm.

Bereits nach 65 min zeigt sich eine deutlich erhöhte UV Absorption der Verbindung im UV-A-Bereich ($E_{max}$ im Bereich 320-340 nm), die bei längerer Bestrahlung weiter zunimmt.

**Beispiel 2a: Oxidation im UV Licht in Gegenwart weiterer Antioxidantien**

Figur 2 zeigt die Veränderung des UV/VIS-Spektrums von Emulsionen enthaltend 0,5 Gew.-% beta-Carotin und 4 Gew.-% 4-Hydroxy-3,5-dimethoxy-benzyl-malonsäure-di-2-ethyl-hexylester (Kurven A und B) im Vergleich zu einer Emulsion enthaltend 0,5 Gew.-% beta-Carotin jedoch kein 4-Hydroxy-3,5-dimethoxy-benzyl-malonsäure-di-2-ethyl-hexylester (Kurven C und D) bei Bestrahlung mit UV-Licht (vgl. Beispiel 2):

Die Kurven stehen für die unbestrahlten Emulsionen (Kurve A und C) und die Emuslionen nach nach 90 min Bestrahlung (Kurven B und D). Die Spektren sind auf einem Carry 50 Spektrometer aufgenommen. Die Bestrahlung erfolgt mittels einer Atlas Sun Test $CPS^+$ Xenon Lampe mit UV-Spezialglas-Filter. Es handelt sich um die Ergebnisse von 4-fach Bestimmungen (n = 4).

Für die bestrahlte 4-Hydroxy-3,5-dimethoxy-benzyl-malonsäure-di-2-ethyl-hexylester enthaltende Probe (B) zeigt sich wieder die Absorption des Reaktionsproduktes im UV-A-Bereich ($E_{max}$ im Bereich 320-340 nm). Darüber hinaus zeigt sich jedoch, dass die Absorption des beta-Carotin ($E_{max}$ im Bereich 440-480 nm) in dieser Probe im Vergleich zu der bestrahlten Probe D deutlich stärker ist. Folglich ist der beta-Carotin Abbau in der erfindungsgemäßen Emulsion reduziert; 4-Hydroxy-3,5-dimethoxybenzyl-malonsäure-di-2-ethyl-hexylester stabilisiert das beta-Carotin.

**Beispiel 2b: DPPH-Assay**

Die radikalreduzierende Wirkung kann beispielsweise mit dem 2,2-Diphenyl-1-picrylhydrazyl(DPPH)-Assay gezeigt werden. 2,2-Diphenyl-1-picrylhydrazyl ist ein in Lösung stabiles freies Radikal. Das ungepaarte Elektron führt zu einer starken Absorptionsbande bei 515 nm, die Lösung ist dunkel-violett gefärbt. In Gegenwart eines Radikalfängers wird das Elektron gepaart, die Absorption verschwindet und die Entfärbung verläuft stöchiometrisch unter Berücksichtigung der aufgenommenen Elektronen. Gemessen wird die Extinktion im Photometer. Die antiradikalische Eigenschaft der zu testenden Substanz wird bestimmt, indem man die Konzentration ermittelt, bei der 50 % des eingesetzten 2,2-Diphenyl-1-picrylhydrazyls mit dem Radikalfänger reagiert haben. Ausgedrückt wird diese Konzentration als $EC_{50}$, ein Wert, der unter den gegebenen Messbedingungen als Substanzeigenschaft zu betrachten ist. Verglichen wird die untersuchte Substanz mit einem Standard (z.B. Tocopherol). Der $EC_{50}$-Wert ist dabei ein Maß für die Kapazität der jeweiligen Verbindung Radikale zu fangen. Je niedriger der $EC_{50}$-Wert ist, desto höher ist die Kapazität Radikale zu fangen.

**Durchführung:**

Es wird eine Stammlösung von 2,2-Diphenyl-1-pikrylhydrozyl (DPPH) in Ethanol hergestellt (0,025 g/L DPPH-Radikale). Aliquots dieser Lösung werden mit verschiedenen Konzentrationen der zu testenden Verbindung versetzt. Es wird bei 515 nm, 25°C und 1 cm jeweils die Extinktion gemessen.
Als $EC_{50}$ wird der Wert ermittelt, bei dem noch 50% der ursprünglichen DPPH-radikal-Konzentration vorliegt. Je kleiner dieser Wert ist, desto höher ist die entsprechende radikalreduzierende Aktivität.
Die Reaktionszeit, die benötigt wird, um diesen Wert zu erreichen wird in dem Wert $T_{EC50}$ angegeben (in Minuten).
In der Tabelle werden Aktivitäten und Stabilitäten einiger gänginger Antioxidantien (bestimmt gemäß dem oben beschriebenen DPPH-assay) den erfindungsgemäßen Antioxidantien gegenübergestellt.

| | Aktivität EC50 [$\mu molL^{-1}$] | Stabilität $T_{EC50}$ [min] |
|---|---|---|
| Hydroxy Dimethoxybenzyl Malonat | 0,30 | 600 |
| Hydroxy Dimethoxybenzyliden Malonat | 6,66 | 1200 |
| Ascorbinsäure | 0,29 | < 5 |
| Ascorbyl (2-O) phosphat | 8,61 | 1200 |
| alpha-Tocopherol | 0,25 | 30 |
| alpha-Tocopheryl acetat | 5040 | 600 |

**Beispiel 3: Zubereitungen**

Im folgenden werden beispielhaft Rezepturen für kosmetische Zubereitungen angegeben, die Verbindungen nach Beispiel 1 enthalten. Entsprechende Zubereitungen können in gleicher Weise mit allen erfindungsgemäßen Verbindungen hergestellt werden.
Im übrigen sind die INCI-Bezeichnungen der handelsüblichen Verbindungen angegeben.
UV-Pearl , OMC steht für die Zubereitung mit der INCI-Bezeichnung:

> Water (for EU: Aqua), Ethylhexyl Methoxycinnamate, Silica, PVP, Chlorphenesin, BHT; diese Zubereitung ist im Handel unter der Bezeichnung Eusolex®UV Pearl™OMC von der Merck KGaA, Darmstadt erhältlich.

Die anderen in den Tabellen angegebenen UV-Pearl sind jeweils analog zusammengesetzt, wobei OMC gegen die angegebenen UV-Filter ausgetauscht ist.

Tabelle 1 W/O-Emulsionen (Zahlen in Gew.-%)

| | 1-1 | 1-2 | 1-3 | 1-4 | 1-5 | 1-6 | 1-7 | 1-8 | 1-9 | 1-10 |
|---|---|---|---|---|---|---|---|---|---|---|
| Titanium dioxide | | 2 | 5 | | | | | | | 3 |
| 4-Hydroxy-3,5-dimethoxy-benzyl-malonsäure-di 2-ethyl-hexylester | 5 | 3 | 2 | 1 | 2 | 1 | 2 | 1 | 1 | 1 |
| Zinc oxide | | | | | | | | 5 | 2 | |
| UV-Pearl , OMC | 30 | 15 | 15 | 15 | 15 | 15 | 15 | 15 | 15 | 15 |
| Polyglyceryl-3-Dimerate | 3 | 3 | 3 | 3 | 3 | 3 | 3 | 3 | 3 | 3 |
| Cera Alba | 0,3 | 0,3 | 0,3 | 0,3 | 0,3 | 0,3 | 0,3 | 0,3 | 0,3 | 0,3 |
| Hydrogenated Castor Oil | 0,2 | 0,2 | 0,2 | 0,2 | 0,2 | 0,2 | 0,2 | 0,2 | 0,2 | 0,2 |
| Paraffinium Liquidum | 7 | 7 | 7 | 7 | 7 | 7 | 7 | 7 | 7 | 7 |
| Caprylic/Capric Triglyceride | 7 | 7 | 7 | 7 | 7 | 7 | 7 | 7 | 7 | 7 |
| Hexyl Laurate | 4 | 4 | 4 | 4 | 4 | 4 | 4 | 4 | 4 | 4 |
| PVP/Eicosene Copolymer | 2 | 2 | 2 | 2 | 2 | 2 | 2 | 2 | 2 | 2 |
| Propylene Glycol | 4 | 4 | 4 | 4 | 4 | 4 | 4 | 4 | 4 | 4 |
| Magnesium Sulfate | 0,6 | 0,6 | 0,6 | 0,6 | 0,6 | 0,6 | 0,6 | 0,6 | 0,6 | 0,6 |
| Tocopherol | 0,5 | 0,5 | 0,5 | 0,5 | 0,5 | 0,5 | 0,5 | 0,5 | 0,5 | 0,5 |
| Tocopheryl Acetate | 0,5 | 0,5 | 0,5 | 0,5 | 0,5 | 0,5 | 0,5 | 0,5 | 0,5 | 0,5 |
| Cyclomethicone | 0,5 | 0,5 | 0,5 | 0,5 | 0,5 | 0,5 | 0,5 | 0,5 | 0,5 | 0,5 |
| Propylparabene | 0,05 | 0,05 | 0,05 | 0,05 | 0,05 | 0,05 | 0,05 | 0,05 | 0,05 | 0,05 |
| Methylparabene | 0,15 | 0,15 | 0,15 | 0,15 | 0,15 | 0,15 | 0,15 | 0,15 | 0,15 | 0,15 |
| Water | ad 100 | ad 100 | ad 100 | ad 100 | ad 100 | ad 100 | ad 100 | ad 100 | ad 100 | ad 100 |

Tabelle 1 (Fortsetzung)

| | 1-11 | 1-12 | 1-13 | 1-14 | 1-15 | 1-16 | 1-17 | 1-18 |
|---|---|---|---|---|---|---|---|---|
| Titanium dioxide | 3 | | 2 | | 3 | | 2 | 5 |
| Benzylidene malonate polysiloxane | | 1 | 0,5 | | | | | |
| 4-Hydroxy-phenyl-propionsäure-2-ethyl-hexylester | 1 | 1 | 0,5 | | | | | |
| 4-Hydroxy-3,5-dimethoxy-benzyl-malonsäure-di 2-ethyl-hexylester | 5 | 3 | 2 | 5 | 1 | 3 | 7 | 2 |
| Polyglyceryl-3-Dimerate | 3 | 3 | 3 | 3 | | | | |
| Cera Alba | 0,3 | 0,3 | 0,3 | 0,3 | 2 | 2 | 2 | 2 |
| Hydrogenated Castor Oil | 0,2 | 0,2 | 0,2 | 0,2 | | | | |
| Paraffinium Liquidum | 7 | 7 | 7 | 7 | | | | |
| Caprylic/Capric Triglyceride | 7 | 7 | 7 | 7 | | | | |
| Hexyl Laurate | 4 | 4 | 4 | 4 | | | | |
| PVP/Eicosene Copolymer | 2 | 2 | 2 | 2 | | | | |
| Propylene Glycol | 4 | 4 | 4 | 4 | | | | |
| Magnesium Sulfate | 0,6 | 0,6 | 0,6 | 0,6 | | | | |
| Tocopherol | 0,5 | 0,5 | 0,5 | 0,5 | | | | |
| Tocopheryl Acetate | 0,5 | 0,5 | 0,5 | 0,5 | 1 | 1 | 1 | 1 |
| Cyclomethicone | 0,5 | 0,5 | 0,5 | 0,5 | | | | |
| Propylparabene | 0,05 | 0,05 | 0,05 | 0,05 | 0,05 | 0,05 | 0,05 | 0,05 |
| Methylparabene | 0,15 | 0,15 | 0,15 | 0,15 | 0,15 | 0,15 | 0,15 | 0,15 |
| Dicocoyl Pentyerythrityl Citrate (and) Sorbitan Sesquioleate (and) Cera Alba (and) Aluminium Stearate | | | | | 6 | 6 | 6 | 6 |
| PEG-7 Hydrogenated Castor Oil | | | | | 1 | 1 | 1 | 1 |
| Zinc Stearate | | | | | 2 | 2 | 2 | 2 |
| Oleyl Erucate | | | | | 6 | 6 | 6 | 6 |
| Decyl Oleate | | | | | 6 | 6 | 6 | 6 |
| Dimethicone | | | | | 5 | 5 | 5 | 5 |
| Tromethamine | | | | | 1 | 1 | 1 | 1 |
| Glycerin | | | | | 5 | 5 | 5 | 5 |
| Allantoin | | | | | 0,2 | 0,2 | 0,2 | 0,2 |
| water | ad 100 | ad 100 | ad 100 | ad 100 | ad 100 | ad 100 | ad 100 | ad 100 |

Tabelle 1 (Fortsetzung)

| | 1-19 | 1-20 | 1-21 | 1-22 | 1-23 | 1-24 | 1-25 | 1-26 | 1-27 | 1-28 | 1-29 |
|---|---|---|---|---|---|---|---|---|---|---|---|
| Titanium dioxide | | 2 | 5 | | | | | | | 3 | 3 |
| Benzylidene malonate polysiloxane | | | | 1 | | | | | 1 | 1 | |
| Methylene Bis-Benztriazolyl Tetramethylbutylphenol | | | | | | 1 | 2 | 1 | | | 1 |
| Zinc oxide | | | | | | | | 5 | 2 | | |
| 4-Hydroxy-phenyl-propionsäure-2-ethyl-hexylester | 5 | 5 | 5 | 5 | 7 | 5 | 5 | 5 | 5 | 5 | 8 |
| UV-Pearl , OCR | | 10 | | | | | | | | | 5 |
| UV-Pearl, EthylhexylDimethylPABA | | | 10 | | | | | | | | |
| 4-Hydroxy-3,5-dimethoxy-benzyl-malonsäure-di 2-ethyl-hexylester | 2 | 4 | 5 | 6 | 3 | 1 | 6 | 10 | 1 | 2 | 5 |
| UV-Pearl, Homosalate, BP-3 | | | | | | | | | 10 | | |
| UV-Pearl, Ethylhexyl salicylate, BP-3 | | | | | | | | | | 10 | |
| BMDBM | | | | | | | | | | | 2 |
| UV-Pearl OMC, 4-Methylbenzylidene Camphor | 25 | | | | | | | | | | |
| Polyglyceryl-3-Dimerate | 3 | 3 | 3 | 3 | 3 | 3 | 3 | 3 | 3 | 3 | 3 |
| Cera Alba | 0,3 | 0,3 | 0,3 | 0,3 | 0,3 | 0,3 | 0,3 | 0,3 | 0,3 | 0,3 | 0,3 |
| Hydrogenated Castor Oil | 0,2 | 0,2 | 0,2 | 0,2 | 0,2 | 0,2 | 0,2 | 0,2 | 0,2 | 0,2 | 0,2 |
| Paraffinium Liquidum | 7 | 7 | 7 | 7 | 7 | 7 | 7 | 7 | 7 | 7 | 7 |
| Caprylic/Capric Triglyceride | 7 | 7 | 7 | 7 | 7 | 7 | 7 | 7 | 7 | 7 | 7 |
| Hexyl Laurate | 4 | 4 | 4 | 4 | 4 | 4 | 4 | 4 | 4 | 4 | 4 |
| PVP/Eicosene Copolymer | 2 | 2 | 2 | 2 | 2 | 2 | 2 | 2 | 2 | 2 | 2 |
| Propylene Glycol | 4 | 4 | 4 | 4 | 4 | 4 | 4 | 4 | 4 | 4 | 4 |
| Magnesium Sulfate | 0,6 | 0,6 | 0,6 | 0,6 | 0,6 | 0,6 | 0,6 | 0,6 | 0,6 | 0,6 | 0,6 |
| Tocopherol | 0,5 | 0,5 | 0,5 | 0,5 | 0,5 | 0,5 | 0,5 | 0,5 | 0,5 | 0,5 | 0,5 |
| Tocopheryl Acetate | 0,5 | 0,5 | 0,5 | 0,5 | 0,5 | 0,5 | 0,5 | 0,5 | 0,5 | 0,5 | 0,5 |
| 3,4-Dihydroxy-phenyl-propionsäure- phenethylester | 0,5 | 0,5 | 0,5 | 0,5 | 0,5 | 0,5 | 0,5 | 0,5 | 0,5 | 0,5 | 0,5 |
| Propylparabene | 0,05 | 0,05 | 0,05 | 0,05 | 0,05 | 0,05 | 0,05 | 0,05 | 0,05 | 0,05 | 0,05 |
| Methylparabene | 0,15 | 0,15 | 0,15 | 0,15 | 0,15 | 0,15 | 0,15 | 0,15 | 0,15 | 0,15 | 0,15 |
| Water | ad 100 | | | | | | | | | | |

Tabelle 2: O/W-Emulsionen, Zahlen in Gew.-%

| | 2-1 | 2-2 | 2-3 | 2-4 | 2-5 | 2-6 | 2-7 | 2-8 | 2-9 | 2-10 |
|---|---|---|---|---|---|---|---|---|---|---|
| Titanium dioxide | | 2 | 5 | | | | | | | 3 |
| Methylene Bis-Benztriazolyl Tetramethylbutylphenol | | | | | | 1 | 2 | 1 | | |
| 3,4-Dihydroxy-phenyl-propionsäure- phenethylester | | | | 1 | 2 | | | | 1 | 1 |
| 4-Hydroxy-phenyl-propionsäure-2-ethyl-hexylester | 1 | 3 | | 2 | | 5 | | 5 | 2 | |
| 4-Hydroxy-3,5-dimethoxy-benzyl-malonsäure-di 2-ethyl-hexylester | 5 | 5 | 5 | 5 | 5 | 5 | 5 | 5 | 5 | 5 |
| 2-Cyano-3,3,-diphenyl--propionsäure -di 2-ethyl-hexylester | 1 | 5 | 4 | | 6 | | 7 | | 2 | 1 |
| 4-Methylbenzyliden Camphor | 2 | | 3 | | 4 | | 3 | | 2 | |
| BMDBM | 1 | 3 | | 3 | 3 | | 3 | 3 | 3 | |
| Stearyl Alcohol (and) Steareth-7 (and) Steareth-10 | 3 | 3 | 3 | 3 | 3 | 3 | 3 | 3 | 3 | 3 |
| Glyceryl Stearate (and) Ceteth-20 | 3 | 3 | 3 | 3 | 3 | 3 | 3 | 3 | 3 | 3 |
| Glyceryl Stearate | 3 | 3 | 3 | 3 | 3 | 3 | 3 | 3 | 3 | 3 |
| Microwax | 1 | 1 | 1 | 1 | 1 | 1 | 1 | 1 | 1 | 1 |
| Cetearyl Octanoate | 11,5 | 11,5 | 11,5 | 11,5 | 11,5 | 11,5 | 11,5 | 11,5 | 11,5 | 11,5 |
| Caprylic/Capric Triglyceride | 6 | 6 | 6 | 6 | 6 | 6 | 6 | 6 | 6 | 6 |
| Oleyl Oleate | 6 | 6 | 6 | 6 | 6 | 6 | 6 | 6 | 6 | 6 |
| Propylene Glycol | 4 | 4 | 4 | 4 | 4 | 4 | 4 | 4 | 4 | 4 |
| Glyceryl Stearate SE | | | | | | | | | | |
| Stearic Acid | | | | | | | | | | |
| Persea Gratissima | | | | | | | | | | |
| Propylparabene | 0,05 | 0,05 | 0,05 | 0,05 | 0,05 | 0,05 | 0,05 | 0,05 | 0,05 | 0,05 |
| Methylparabene | 0,15 | 0,15 | 0,15 | 0,15 | 0,15 | 0,15 | 0,15 | 0,15 | 0,15 | 0,15 |
| Tromethamine | | | 1,8 | | | | | | | |
| Water | ad 100 | ad 100 | ad 100 | ad 100 | ad 100 | ad 100 | ad 100 | ad 100 | ad 100 | ad 100 |

Tabelle 2 (Fortsetzung) *nicht erfindungsgemäß

| | 2-11* | 2-12* | 2-13* | 2-14 | 2-15 | 2-16* | 2-17* | 2-18* |
|---|---|---|---|---|---|---|---|---|
| Titanium dioxide | 3 | | 2 | | | | 2 | 5 |
| Benzylidene malonate polysiloxane | | 1 | 0,5 | | | | | |
| 3,4-Dihydroxy-phenyl-propionsäure- phenethylester | 1 | 1 | 0,5 | | | | | |
| 4-Hydroxy-3,5-dimethoxy-benzyl-malonsäure-di 2-ethyl-hexylester | | | | 1 | 2 | | | |
| 2-Cyano-3,3,-diphenyl--propionsäure -di 2-ethyl-hexylester | 1 | 3 | | 2 | | 5 | | 5 |
| 5,6,7-Trihydroxyflavon | 5 | 5 | 5 | 5 | 5 | 5 | 5 | 5 |
| 4-Hydroxy-phenyl-propionsäure-2-ethyl-hexylester | 1 | 5 | 4 | | 6 | | 7 | |
| Zinc oxide | | | 2 | | | | | |
| UV-Pearl , OMC | 15 | 15 | 15 | 30 | 30 | 30 | 15 | 15 |
| 4-Methylbenzyliden Camphor | | | | 3 | | | | |
| BMDBM | | | | 1 | | | | |
| Phenylbenzimidazole Sulfonic Acid | | | | | 4 | | | |
| Stearyl Alcohol (and) Steareth-7 (and) Steareth-10 | 3 | 3 | 3 | 3 | | | | |
| Glyceryl Stearate (and) Ceteth-20 | 3 | 3 | 3 | 3 | | | | |
| Glyceryl Stearate | 3 | 3 | 3 | 3 | | | | |
| Microwax | 1 | 1 | 1 | 1 | | | | |
| Cetearyl Octanoate | 11,5 | 11,5 | 11,5 | 11,5 | | | | |
| Caprylic/Capric Triglyceride | 6 | 6 | 6 | 6 | 14 | 14 | 14 | 14 |
| Oleyl Oleate | 6 | 6 | 6 | 6 | | | | |
| Propylene Glycol | 4 | 4 | 4 | 4 | | | | |
| Glyceryl Stearate SE | | | | | 6 | 6 | 6 | 6 |
| Stearic Acid | | | | | 2 | 2 | 2 | 2 |
| Persea Gratissima | | | | | 8 | 8 | 8 | 8 |
| Propylparabene | 0,05 | 0,05 | 0,05 | 0,05 | 0,05 | 0,05 | 0,05 | 0,05 |
| Methylparabene | 0,15 | 0,15 | 0,15 | 0,15 | 0,15 | 0,15 | 0,15 | 0,15 |
| Tromethamine | | | | | 1,8 | | | |
| Glycerin | | | | | 3 | 3 | 3 | 3 |
| Water | ad 100 | ad 100 | ad 100 | ad 100 | ad 100 | ad 100 | ad 100 | ad 100 |

Tabelle 2 (Fortsetzung) *nicht erfindungsgemäß

| | 2-19 | 2-20 | 2-21 | 2-22* | 2-23 | 2-24* | 2-25 | 2-26* | 2-27 | 2-28 |
|---|---|---|---|---|---|---|---|---|---|---|
| Titanium dioxide | | | | | | | 3 | 3 | | 2 |
| Benzylidene malonate polysiloxane | 1 | 2 | | | | 1 | 1 | | 1 | 0,5 |
| 7,8,3',4'-Tetrahydroxyflavon | | | | 1 | 2 | | | | 1 | 1 |
| 4-Hydroxy-phenyl-propionsäure-2-ethyl-hexylester | 1 | 3 | | 2 | | 5 | | 5 | 2 | |
| 2-Cyano-3,3,-diphenyl--propionsäure -di 2-ethyl-hexylester | 5 | 5 | 5 | 5 | 5 | 5 | 5 | 5 | 5 | 5 |
| 4-Hydroxy-3,5-dimethoxy-benzyl-malonsäure-di 2-ethyl-hexylester | 1 | 5 | 4 | | 6 | | 7 | | 2 | 1 |
| 3,4-Dihydroxy-phenyl-propionsäure- phenethylester | | | 1 | 2 | 1 | | | 1 | 1 | 0,5 |
| Zinc oxide | | | | | 5 | 2 | | | | 2 |
| UV-Pearl , OMC | 15 | 15 | 15 | 15 | 15 | 15 | 15 | 15 | 15 | 15 |
| Caprylic/Capric Triglyceride | 14 | 14 | 14 | 14 | 14 | 14 | 14 | 14 | 14 | 14 |
| Oleyl Oleate | | | | | | | | | | |
| Propylene Glycol | | | | | | | | | | |
| Glyceryl Stearate SE | 6 | 6 | 6 | 6 | 6 | 6 | 6 | 6 | 6 | 6 |
| Stearic Acid | 2 | 2 | 2 | 2 | 2 | 2 | 2 | 2 | 2 | 2 |
| Persea Gratissima | 8 | 8 | 8 | 8 | 8 | 8 | 8 | 8 | 8 | 8 |
| Propylparabene | 0,05 | 0,05 | 0,05 | 0,05 | 0,05 | 0,05 | 0,05 | 0,05 | 0,05 | 0,05 |
| Methylparabene | 0,15 | 0,15 | 0,15 | 0,15 | 0,15 | 0,15 | 0,15 | 0,15 | 0,15 | 0,15 |
| Glyceryl Stearate, Ceteareth-20, Ceteareth-10, Cetearyl Alcohol, Cetyl Palmitate | | | | | | | | | | |
| Ceteareth-30 | | | | | | | | | | |
| Dicaprylyl Ether | | | | | | | | | | |
| Glycerin | 3 | 3 | 3 | 3 | 3 | 3 | 3 | 3 | 3 | 3 |
| Water | ad 100 | ad 100 | ad 100 | ad 100 | ad 100 | ad 100 | ad 100 | ad 100 | ad 100 | ad 100 |

Tabelle 3: Gele, Zahlen in Gew.-% *nicht erfindungsgemäß

| | 3-1 | 3-2 | 3-3* | 3-4 | 3-5* | 3-6 | 3-7* | 3-8 | 3-9 | 3-10* |
|---|---|---|---|---|---|---|---|---|---|---|
| A = aqueous gel | | | | | | | | | | |
| Titanium dioxide | | 2 | 5 | | | | | | | 3 |
| 5,6,7-Trihydroxyflavon | | | | 1 | 2 | | | | 1 | 1 |
| 4-Hydroxy-3,5-dimethoxy-benzyl-malonsäure-di 2-ethyl-hexylester | 1 | 3 | | 2 | | 5 | | 5 | 2 | |
| 2-Cyano-3,3,-diphenyl--propionsäure -di 2-ethyl-hexylester | 5 | 5 | 5 | 5 | 5 | 5 | 5 | 5 | 5 | 5 |
| 4-Hydroxy-phenyl-propionsäure-2-ethyl-hexylester | 1 | 5 | 4 | | 6 | | 7 | | 2 | 1 |
| Benzylidene malonate polysiloxane | | | 1 | 1 | 2 | | | | 1 | 1 |
| Methylene Bis-Benztriazolyl Tetramethylbutylphenol | | 1 | | | | 1 | 2 | 1 | | |
| Zinc oxide | | | | 2 | | | | 5 | 2 | |
| UV-Pearl , Ethylhexyl Mehtoxycinnamat | 30 | 15 | 15 | 15 | 15 | 15 | 15 | 15 | 15 | 15 |
| 4-Methylbenzyliden Camphor | | | | | 2 | | | | | |
| Butylmethoxydibenzoylmethane | | 1 | | | | | | | | |
| Phenylbenzimidazole Sulfonic Acid | | | 4 | | | | | | | |
| Prunus Dulcis | 5 | 5 | 5 | 5 | 5 | 5 | 5 | 5 | 5 | 5 |
| Tocopheryl Acetate | 0,5 | 0,5 | 0,5 | 0,5 | 0,5 | 0,5 | 0,5 | 0,5 | 0,5 | 0,5 |
| Caprylic/Capric Triglyceride | 3 | 3 | 3 | 3 | 3 | 3 | 3 | 3 | 3 | 3 |
| Octyldodecanol | 2 | 2 | 2 | 2 | 2 | 2 | 2 | 2 | 2 | 2 |
| Decyl Oleate | 2 | 2 | 2 | 2 | 2 | 2 | 2 | 2 | 2 | 2 |
| PEG-8 (and) Tocopherol (and) Ascorbyl Palmitate (and) Ascorbic Acid (and) Citric Acid | 0,05 | 0,05 | 0,05 | 0,05 | 0,05 | 0,05 | 0,05 | 0,05 | 0,05 | 0,05 |
| Sorbitol | 4 | 4 | 4 | 4 | 4 | 4 | 4 | 4 | 4 | 4 |
| Polyacrylamide (and) C13-14 Isoparaffin (and) Laureth-7 | 3 | 3 | 3 | 3 | 3 | 3 | 3 | 3 | 3 | 3 |
| Propylparabene | 0,05 | 0,05 | 0,05 | 0,05 | 0,05 | 0,05 | 0,05 | 0,05 | 0,05 | 0,05 |
| Methylparabene | 0,15 | 0,15 | 0,15 | 0,15 | 0,15 | 0,15 | 0,15 | 0,15 | 0,15 | 0,15 |
| Tromethamine | | | 1,8 | | | | | | | |
| Water | ad 100 | ad 100 | ad 100 | ad 100 | ad 100 | ad 100 | ad 100 | ad 100 | ad 100 | ad 100 |

Tabelle 3 (Fortsetzung) *nicht erfindungsgemäß

| | 3-11* | 3-12* | 3-13* | 3-14 | 3-15 | 3-16* | 3-17* | 3-18* |
|---|---|---|---|---|---|---|---|---|
| a = aqueaous gel | | | | A | a | a | a | a |
| Titanium dioxide | 3 | | 2 | | | | | |
| Benzylidene malonate polysiloxane | | 1 | 0,5 | 1 | 2 | | | |
| Methylene Bis-Benztriazolyl Tetramethylbutylphenol | 1 | 1 | 0,5 | | | 1 | 2 | 1 |
| 4-Hydroxy-3,5-dimethoxy-benzyl-malonsäure-di 2-ethyl-hexylester | | | | 1 | 2 | | | |
| 4-Hydroxy-phenyl-propionsäure-2-ethyl-hexylester | 1 | 3 | | 2 | | 5 | | 5 |
| 2-Cyano-3,3,-diphenyl--propionsäure - di 2-ethyl-hexylester | 5 | 5 | 5 | 5 | 5 | 5 | 5 | 5 |
| 6,3',4'-Trihydroxyflavon | 1 | 5 | 4 | | 6 | | 7 | |
| Zinc oxide | | | 2 | | | | | |
| UV-Pearl , Ethylhexyl Mehtoxycinnamat | 15 | 15 | 15 | 15 | 15 | 15 | 15 | 15 |
| Prunus Dulcis | 5 | 5 | 5 | | | | | |
| Tocopheryl Acetate | 0,5 | 0,5 | 0,5 | | | | | |
| Caprylic/Capric Triglyceride | 3 | 3 | 3 | | | | | |
| Octyldodecanol | 2 | 2 | 2 | | | | | |
| Decyl Oleate | 2 | 2 | 2 | | | | | |
| PEG-8 (and) Tocopherol (and) Ascorbyl Palmitate (and) Ascorbic Acid (and) Citric Acid | 0,05 | 0,05 | 0,05 | | | | | |
| Sorbitol | 4 | 4 | 4 | 5 | 5 | 5 | 5 | 5 |
| Polyacrylamide (and) C13-14 Isoparaffin (and) Laureth-7 | 3 | 3 | 3 | | | | | |
| Carbomer | | | | 1,5 | 1,5 | 1,5 | 1,5 | 1,5 |
| Propylparabene | 0,05 | 0,05 | 0,05 | | | | | |
| Methylparabene | 0,15 | 0,15 | 0,15 | 0,15 | 0,15 | 0,15 | 0,15 | 0,15 |
| Allantoin | | | | 0,2 | 0,2 | 0,2 | 0,2 | 0,2 |
| Tromethamine | | | | 2,4 | 2,4 | 2,4 | 2,4 | 2,4 |
| Water | ad 100 | ad 100 | ad 100 | ad 100 | ad 100 | ad 100 | ad 100 | ad 100 |

Tabelle 3 (Fortsetzung) *nicht erfindungsgemäß

| | 3-19 | 3-20 | 3-21* | 3-22 | 3-23* | 3-24 | 3-25* | 3-26 | 3-27 | 3-28* |
|---|---|---|---|---|---|---|---|---|---|---|
| 7,8,3',4'-Tetrahydroxyflavon | | | | 1 | 2 | | | | 1 | 1 |
| 4-Hydroxy-3,5-dimethoxy-benzyl-malonsäure-di 2-ethyl-hexylester | 1 | 3 | | 2 | | 5 | | 5 | 2 | |
| 2-Cyano-3,3,-diphenyl--propionsäure -di 2-ethyl-hexylester | 5 | 5 | 5 | 5 | 5 | 5 | 5 | 5 | 5 | 5 |
| 4-Hydroxy-phenyl-propionsäure-2-ethyl-hexylester | 1 | 5 | 4 | | 6 | | 7 | | 2 | 1 |
| UV-Pearl , OMC | 30 | 30 | 15 | 15 | 15 | 11 | 12 | 15 | 15 | 15 |
| Phenylbenzimidazole Sulfonic Acid | | 4 | 4 | | | | | | | |
| Sorbitol | 5 | 5 | 5 | 5 | 5 | 5 | 5 | 5 | 5 | 5 |
| Carbomer | 1,5 | 1,5 | 1,5 | 1,5 | 1,5 | 1,5 | 1,5 | 1,5 | 1,5 | 1,5 |
| Propylparabene | | | | | | | | | | |
| Methylparabene | 0,15 | 0,15 | 0,15 | 0,15 | 0,15 | 0,15 | 0,15 | 0,15 | 0,15 | 0,15 |
| Allantoin | 0,2 | 0,2 | 0,2 | 0,2 | 0,2 | 0,2 | 0,2 | 0,2 | 0,2 | 0,2 |
| Tromethamine | 2,4 | 4,2 | 4,2 | 2,4 | 2,4 | 2,4 | 2,4 | 2,4 | 2,4 | 2,4 |
| Water | ad 100 | ad 100 | ad 100 | ad 100 | ad 100 | ad 100 | Ad 100 | ad 100 | ad 100 | ad 100 |

Tabelle 3 (Fortsetzung)

| | 3-29 | 3-30 | 3-31 | 3-32 | 3-33 | 3-34 | 3-35 | 3-36 |
|---|---|---|---|---|---|---|---|---|
| 4-Hydroxy-phenyl-propionsäure-2-ethyl-hexylester | | | | 1 | 2 | | | |
| 2-Cyano-3,3,-diphenyl--propionsäure -di 2-ethyl-hexylester | 1 | 3 | | 2 | | 5 | | 5 |
| 4-Hydroxy-3,5-dimethoxy-benzyl-malonsäure-di 2-ethyl-hexylester | 5 | 5 | 5 | 5 | 5 | 5 | 5 | 5 |
| 5,6,7-Trihydroxyflavon | 1 | 5 | 4 | | 6 | | 7 | |
| UV-Pearl , OMC | 15 | 10 | | 10 | 10 | 10 | 15 | 10 |
| UV-Pearl , OCR | | | 10 | | | | | |
| UV-Pearl , OMC, Methylene Bis-Benztriazolyl Tetramethylbutylphenol | | 7 | | 6 | | | | |
| UV-Pearl, Ethylhexyl salicylate, BMDBM | | | 10 | | | | | |
| Disodium Phenyl Dibenzimidazole Tetrasulfonate | | 3 | | | | 3 | | 3 |
| Phenylbenzimidazole Sulfonic Acid | | 2 | | | 2 | 3 | | 3 |
| Prunus Dulcis | 5 | 5 | 5 | | | | | |
| Tocopheryl Acetate | 0,5 | 0,5 | 0,5 | | | | | |
| Caprylic/Capric Triglyceride | 3 | 3 | 3 | | | | | |
| Octyldodecanol | 2 | 2 | 2 | | | | | |
| Decyl Oleate | 2 | 2 | 2 | | | | | |
| PEG-8 (and) Tocopherol (and) Ascorbyl Palmitate (and) Ascorbic Acid (and) Citric Acid | 0,05 | 0,05 | 0,05 | | | | | |
| Sorbitol | 4 | 4 | 4 | 5 | 5 | 5 | 5 | 5 |
| Polyacrylamide (and) C13-14 Isoparaffin (and) Laureth-7 | 3 | 3 | 3 | | | | | |
| Carbomer | | | | 1,5 | 1,5 | 1,5 | 1,5 | 1,5 |
| Propylparabene | 0,05 | 0,05 | 0,05 | | | | | |
| Methylparabene | 0,15 | 0,15 | 0,15 | 0,15 | 0,15 | 0,15 | 0,15 | 0,15 |
| Allantoin | | | | 0,2 | 0,2 | 0,2 | 0,2 | 0,2 |
| Tromethamine | | | | 2,4 | 2,4 | 2,4 | 2,4 | 2,4 |
| Water | ad 100 | ad 100 | ad 100 | ad 100 | ad 100 | ad 100 | ad 100 | ad 100 |

**Verzeichnis der Figuren**

**Figuren 1a und 1b:**

Figur 1 (Figur 1b stellt einen Ausschnitt der Figur 1a dar.) zeigt die Veränderung des UV-Spektrums von 4-Hydroxy-3,5-dimethoxy-benzyl-malonsäure-di 2-ethyl-hexylester bei Bestrahlung mit UV-Licht (vgl. Beispiel 3): Die Kurven stehen für die unbestrahlte Substanz (0 kJ/$m^2$ eingestrahlt), nach 15 min Bestrahlung (86 kJ/$m^2$ eingestrahlt), nach 65 min Bestrahlung (373 kJ/$m^2$ eingestrahlt), nach 235 min Bestrahlung (1349 kJ/$m^2$ eingestrahlt) und nach 405 min Bestrahlung (2325 kJ/$m^2$ eingestrahlt). Die Spektren sind auf einem Cary 300 bio Spektrometer aufgenommen. Die Bestrahlung erfolgt mittels einer Atlas Sun Test CPS, Xenon Lamp mit UV-Spezialglas-Filter bei einer Leistung von 95,69 W/$m^2$ im Bereich 290 - 400 nm. Es handelt sich um die Ergebnisse von 4-fach Bestimmungen (n = 4).

**Figur 2:**

Figur 2 zeigt die Veränderung des UV/VIS-Spektrums von Emulsionen enthaltend 0,5 Gew.-% beta-Carotin und 4 Gew.-% 4-Hydroxy-3,5-dimethoxy-benzyl-malonsäure-di-2-ethyl-hexylester (Kurven A und B) im Vergleich zu einer Emulsion enthaltend 0,5 Gew.-% beta-Carotin jedoch kein 4-Hydroxy-3,5-dimethoxy-benzyl-malonsäure-di-2-ethyl-hexylester (Kurven C und D) bei Bestrahlung mit UV-Licht (vgl. Beispiel 2a): Die Kurven stehen für die unbestrahlten Emulsionen (Kurve A und C) und die Emulsionen nach 90 min Bestrahlung (Kurven B und D). Die Spektren sind auf einem Cary 300 bio Spektrometer aufgenommen. Die Bestrahlung erfolgt mittels einer Atlas Sun Test CPS, Xenon Lamp mit UV-Spezialglas-Filter bei einer Leistung von 95,69 W/$m^2$ im Bereich 290 - 400 nm. Es handelt sich um die Ergebnisse von 4-fach Bestimmungen (n = 4).

## Patentansprüche

1. 4-Hydroxy-3,5-dimethoxy-benzyl-malonsäure-di-2-ethylhexylester.

2. Verfahren zur Herstellung der Verbindung nach Anspruch 1, **dadurch gekennzeichnet, dass** 4-Hydroxy-3,5-dimethoxy-benzyliden-malonsäure-di-2-ethylhexylester hydriert wird.

3. Nicht-therapeutische Verwendung der Verbindung nach Anspruch 1 als Antioxidans.

4. Verwendung der Verbindung nach Anspruch 1 als Antioxidans zur Herstellung kosmetischer oder dermatologischer Zubereitungen oder zur Herstellung von Haushaltsprodukten.

5. Verwendung der Verbindung nach Anspruch 1 zum Produktschutz.

6. Verwendung nach Anspruch 5 zum Schutz organischer oder anorganischer Farbstoffe, Antioxidantien, Vitamine, Parfumkomponenten, Ölkomponenten oder Matrixbestandteile, wie Emulgatoren, Verdicker, Filmbildner und Tenside.

## Claims

1. Di-2-ethylhexyl 4-hydroxy-3,5-dimethoxybenzylmalonate.

2. Process for the preparation of the compound according to Claim 1, **characterised in that** di-2-ethylhexyl 4-hydroxy-3,5-dimethoxy-benzylidenemalonate is hydrogenated.

3. Non-therapeutic use of the compound according to Claim 1 as antioxidant.

4. Use of the compound according to Claim 1 as antioxidant for the preparation of cosmetic or dermatological preparations or for the production of household products.

5. Use of the compound according to Claim 1 for product protection.

6. Use according to Claim 5 for the protection of organic or inorganic dyes, antioxidants, vitamins, perfume components,

oil components or matrix constituents, such as emulsifiers, thickeners, film formers and surfactants.

## Revendications

1. 4-Hydroxy-3,5-diméthoxybenzylmalonate de di-2-éthylhexyle.

2. Procédé de préparation du composé selon la revendication 1, **caractérisé en ce que** du 4-hydroxy-3,5-diméthoxybenzylidène-malonate de di-2-éthylhexyle est hydrogéné.

3. Utilisation non thérapeutique du composé selon la revendication 1 comme antioxydant.

4. Utilisation du composé selon la revendication 1 comme antioxydant, pour la préparation de préparations cosmétiques ou dermatologiques ou pour la production de produits ménagers.

5. Utilisation du composé selon la revendication 1, pour la protection de produits.

6. Utilisation selon la revendication 5, pour la protection de colorants organiques ou inorganiques, d'antioxydants, de vitamines, de composants de parfum, de composants d'huile ou de constituants de matrice, tels que des émulsifiants, des épaississants, des agents filmogènes et des agents tensioactifs.

Fig. 1a:

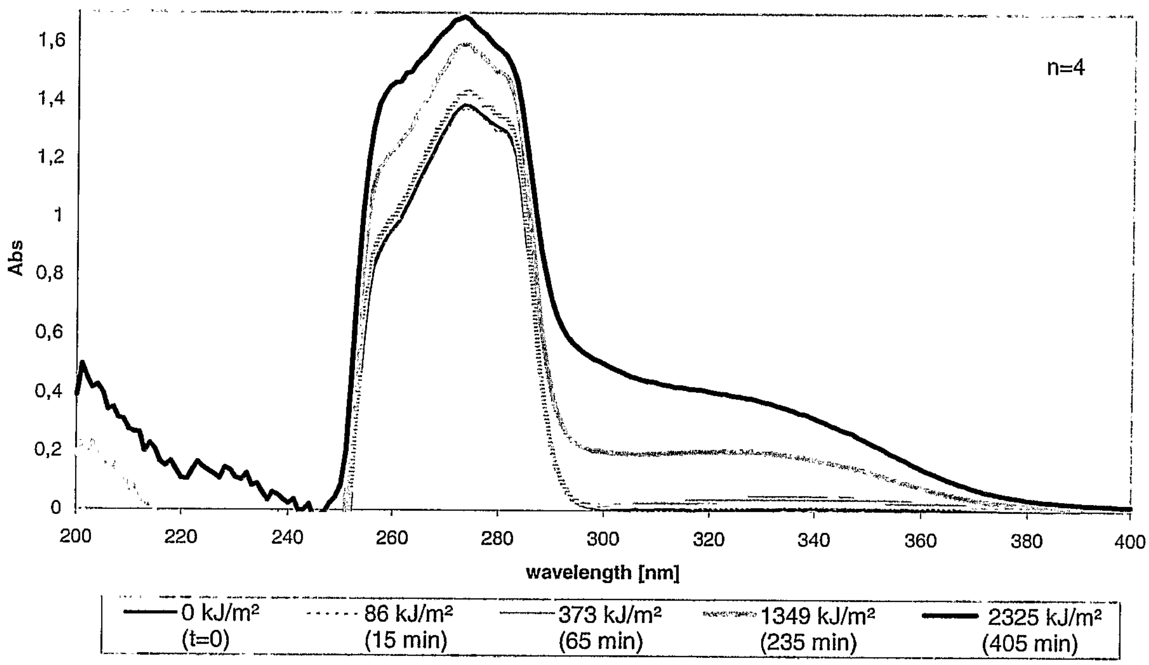

n=4
Abs
1,6
1,4
1,2
1
0,8
0,6
0,4
0,2
0
200
220
240
260
280
300
320
340
360
380
400
wavelength [nm]
0 kJ/m² (t=0)
86 kJ/m² (15 min)
373 kJ/m² (65 min)
1349 kJ/m² (235 min)
2325 kJ/m² (405 min)

Fig. 1b:

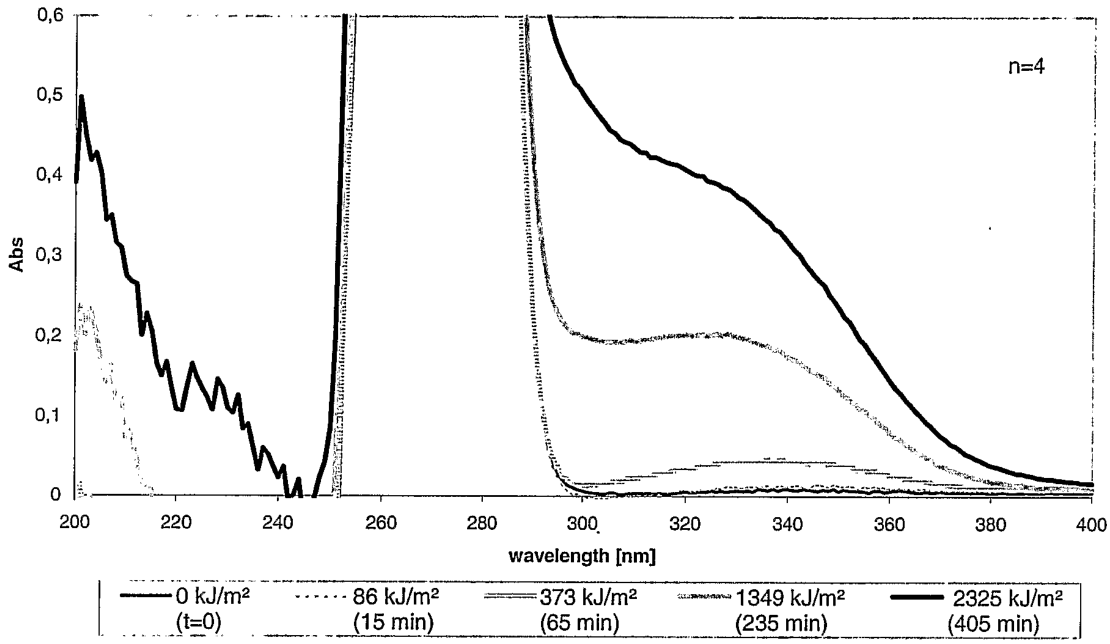
0,6
0,5
0,4
0,3
0,2
0,1
0
Abs
n=4
200
220
240
260
280
300
320
340
360
380
400
wavelength [nm]
0 kJ/m² (t=0)
86 kJ/m² (15 min)
373 kJ/m² (65 min)
1349 kJ/m² (235 min)
2325 kJ/m² (405 min)

Fig. 2

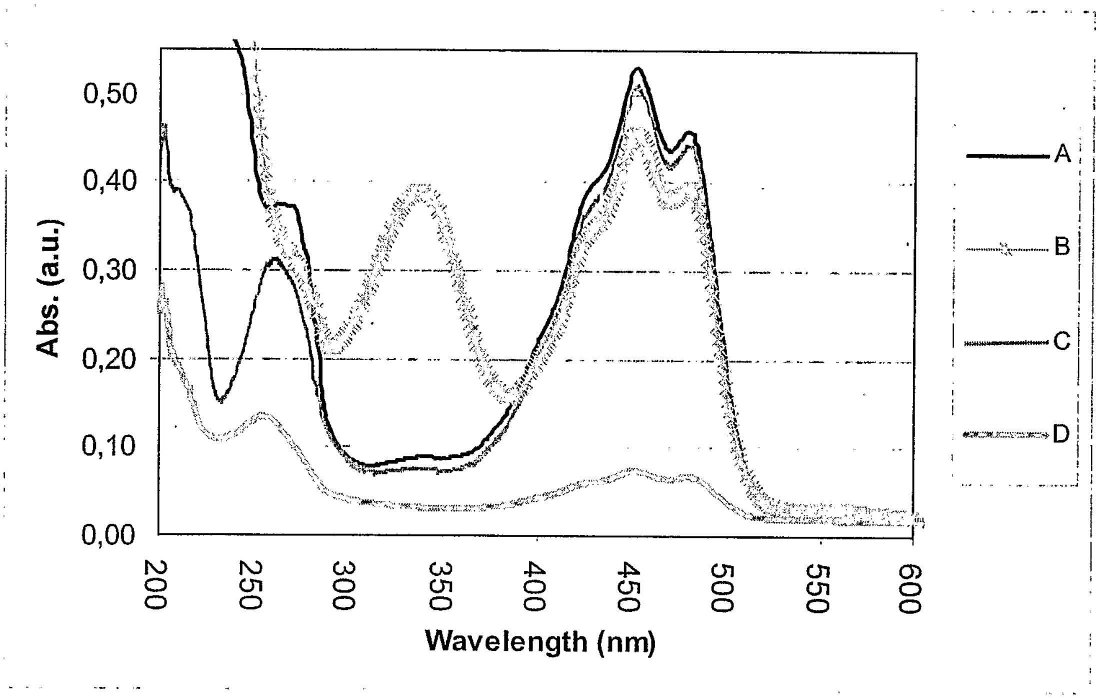
0,50
0,40
0,30
0,20
0,10
0,00
Abs. (a.u.)
200
250
300
350
400
450
500
550
600
Wavelength (nm)
A
B
C
D

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- WO 03007906 A
- JP 01013017 A
- EP 0100651 A
- US 5175340 A
- US 6242099 B1
- WO 0009652 A
- WO 0072806 A
- WO 0071084 A
- EP 0671161 A
- DE 4116123 A
- DE 4308282 A
- WO 2003007906 A

### In der Beschreibung aufgeführte Nicht-Patentliteratur

- Laut CD Römpp Chemie Lexikon - Version 1.0. Georg Thieme Verlag, 1995
- **C. SANCHEZ-MORENO ; J.A. LARRAURI ; F. SAURA-CALIXTO.** *J. Sci. Food Agric.,* 1998, vol. 76 (2), 270-276
- **E. A. GALINSKI et al.** *Eur. J. Biochem.,* 1985, vol. 149, 135-139